# EUROPEAN PATENT APPLICATION

(11) **EP 0 934 932 A1**
(43) Date of publication of application: **11.08.1999**
(21) Application number: 97936858.6
(22) Date of filing: 22.08.1997
(51) Int. Cl.: C07D 215/22, C07D 401/06, C07D 405/12, C07D 405/14, C07D 413/06, C07D 413/12, C07D 417/06, A61K 31/495, A61K 31/535, A61K 31/54

(54) **QUINOLINE DERIVATIVES AND PSYCHOTROPIC AGENT**

(30) Priority: 22.08.1996 JP 22100396
(71) Applicant: MEIJI SEIKA KABUSHIKI KAISHA, Tokyo 104 (JP)
(72) Inventor: HASEGAWA, Toshifumi, Pharm. Res. Center, Kouhoku-ku, Yokohama-shi, Kanagawa 222 (JP); SATO, Eriko, Pharmaceutical Research Center, Kouhoku-ku, Yokohama-shi, Kanagawa 222 (JP); AKIYAMA, Yoshihisa, Pharmaceutical Research Center, Kouhoku-ku, Yokohama-shi, Kanagawa 222 (JP); MORI, Tomohisa, Pharmaceutical Research Center, Kouhoku-ku, Yokohama-shi, Kanagawa 222 (JP); YAMAUCHI, Miki, Pharmaceutical Research Center, Kouhoku-ku. Yokohama-shi, Kanagawa 222 (JP); IMANISHI, Taiichiro, Pharmaceutical Res. Center, Kouhoku-ku, Yokohama-shi, Kanagawa 222 (JP); IMAI, Takahiro, Pharmaceutical Res. Center, Kouhoku-ku, Yokohama-shi, Kanagawa 222 (JP); KUBOTA, Dai, Pharmaceutical Res. Center, Kouhoku-ku, Yokohama-shi, Kanagawa 222 (JP)
(74) Representative: Hall, Marina
(86) International application number: JP9702925
(87) International publication number: WO9807703

(57) **Abstract**

A psychotropic pharmaceutical composition comprising a compound represented by general formula (I) or a pharmacologically acceptable salt or solvate thereof: 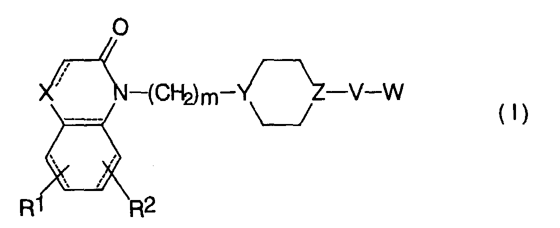 wherein m is an integer of 1 to 4, R¹ and R² represent a substituent, X represents CH, CH₂, O, S, SO, or S O₂, Y and Z each independently represent CH or N, V represents O or -(CH₂)ₙ- (wherein n is an integer of 1 to 4), and W represents a group selected from the group consisting of groups (i) to (iii): 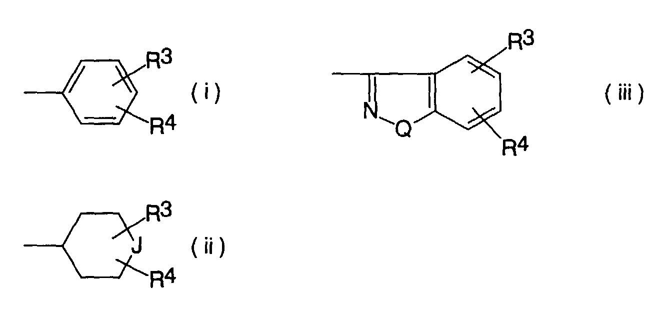 wherein J represents CH₂ or O, Q represents O, S, or NH, and R³ and R⁴ represent a substituent.

## Description

### BACKGROUND OF THE INVENTION

### Field of the Invention

The present invention relates to quinolinone derivatives with psychotropic activity and psychotropic agents comprising the same as an active ingredient.

Blockers for dopamine D₂ receptors such as chlorpromazine and haloperidol have been used as antipsychotic agents. The blockers are effective in treating positive symptoms of schizophrenia, but are not effective in treating negative symptoms thereof. Furthermore, they are known to cause extrapyramidal side effects by blocking dopamine D₂ receptors of corpus striatum.

Advance of the molecular biology of dopamine receptors led to the discovery of subtypes of D₁ to D₅ dopamine receptors. Among them, the dopamine D₄ receptors (Van Tol, et al., Nature, 350, 610 (1991)) are mainly found in frontal cortex that is thought to be deeply involved in schizophrenia, while they have less density in corpus striatum that is related to side effects. It has also been reported that, the density of dopamine D₄ receptors is elevated in patients suffering from schizophrenia, comparing with healthy persons (Seeman. P., et al., Nature, 365, 441 (1993)). Clozapine, which is effective in treating negative symptoms and has no significant extrapyramidal side effects, has been found to bind to dopamine D₄ receptors. It has been reported that the therapeutic concentration of chlozapine acts primarily at dopamine D₄ receptors rather than dopamine D₂ receptors (Seeman P., Neuropsychopharmacology, 7, 261 (1992)).

On the other hand, quinolinone derivatives having allylpiperazine, allylpiperidine, benzylpiperazine, and benzylpiperidine structures are disclosed in Japanese Patent Laid-Open No. 169569/1990, WO94/18197, Japanese Patent Laid-Open No.33744/1995, EP/577325, Japanese Patent Laid-Open No.40946/1994, EP/512525, EP/470514, Japanese Patent Laid-Open Nos. 264773/1990, 169569/1990, and 290821/1988, EP/296560, Japanese Patent Laid-Open Nos. 146872/1988, and 49359/1981, German Patent Publication No. 2009685, Arch. Pharm., 8 (327), 529-31, (1994), Drug Des. Discovery, 11 (3), 197-203, (1994), Med.Chem.Res., 2 (2), 82-7, (1992), and J. Med. Chem., 12 (4), 580-3, (1969). However, affinity of quinolinone derivatives for dopamine D₄ receptors has not been reported yet.

### SUMMARY OF THE INVENTION

The present inventors have searched for novel quinolinone derivatives that have no significant extrapyramidal side effects and have binding affinity for dopamine D₄ receptors. As a result, a group of quinolinone derivatives has been found to have affinity for dopamine D₄ receptors. The present invention has been made based on these findings.

According to one aspect of the present invention, there is provided a psychotropic pharmaceutical composition comprising a compound represented by general formula (I) or a pharmaceutically acceptable salt or solvate thereof: 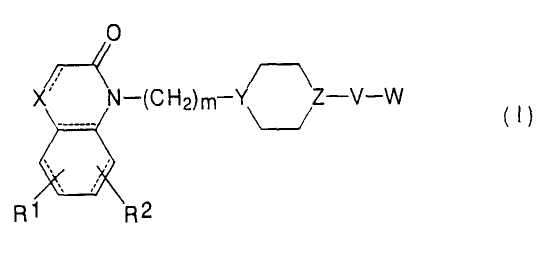 wherein
a solid line with a dotted line represents a single bond or a double bond;
m represents an integer of 1 to 4;
R¹ and R², which may be the same or different, represent a hydrogen atom, a halogen atom, hydroxy, cyano, nitro, trifluoromethyl, -ORa, -SRa, -SORa, -SO₂NRaRb, -NRaRb, -NRaCORb, -NRaCOORb, -CORa, -COORa, or lower alkyl optionally substituted by a halogen atom, wherein Ra and Rb, which may be the same or different, represent lower alkyl optionally substituted by a hydrogen atom or a halogen atom;
X represents CH, CH₂, O, S, SO, or SO₂;
Y and Z, which may be the same or different, represent CH or N;
V represents O or -(CH₂)ₙ- wherein n is an integer of 1 to 4; and
W represents a group selected from the group consisting of groups (i) to (iii): 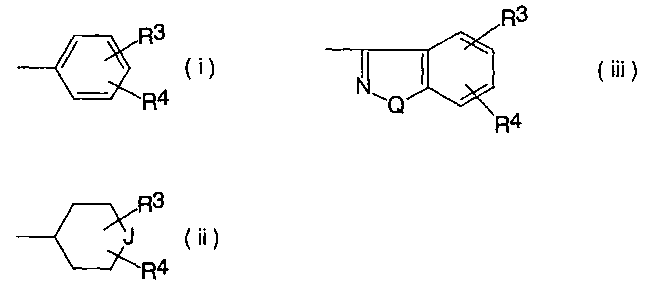 wherein J represents CH₂ or O, Q represents O, S, or NH, and R³ and R⁴, which may be the same or different, represent a hydrogen atom, a halogen atom, cyano, or lower alkyl or lower alkoxy optionally substituted by a halogen atom, or R³ and R⁴ may form a five- or six-membered saturated or unsaturated ring which may contain one or more oxygen, nitrogen, or sulfr atoms together with a carbon atom to which they are attached.

According to another aspect of the present invention, there is provided a novel compound of general formula (Ia): 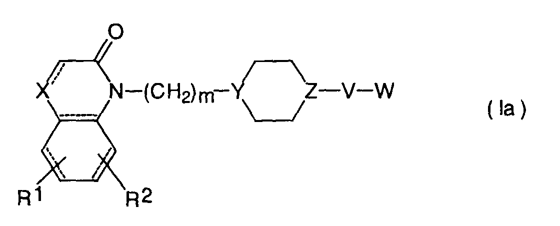 wherein
a solid line with a dotted line represents a single bond or a double bond;
m is an integer of 1 to 4;
R¹ and R², which may be the same or different, represent a hydrogen atom, a halogen atom, hydroxy, cyano, nitro, trifluoromethyl, -ORa, -SRa, -SORa, -SO₂NRaRb, -NRaRb, -NRaCORb, -NRaCOORb, -CORa, -COORa, or lower alkyl optionally substituted by a halogen atom, wherein Ra and Rb, which may be the same or different, represent hydrogen or lower alkyl optionally substituted by a halogen atom;
X represents CH, CH₂, O, S, SO, or SO₂;
Y and Z, which may be the same or different, represent CH or N;
V represents O or -(CH₂)ₙ- wherein n is an integer of 1 to 4; and
W represents a group selected from the group consisting of groups (i) to (iii): 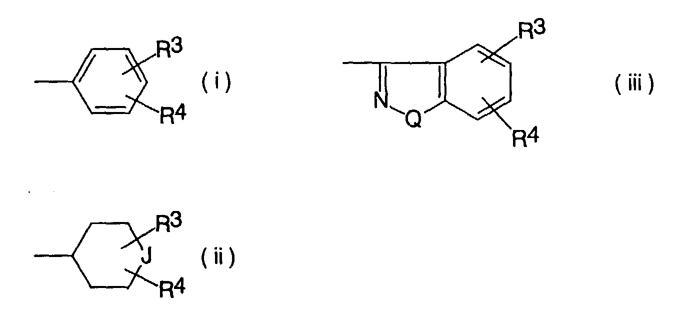 wherein J represents CH₂ or O, Q represents O, S, or NH, and R³ and R⁴, which may be the same or different, represent a hydrogen atom, a halogen atom, cyano, or lower alkyl or lower alkoxy optionally substituted by a halogen atom, or R³ and R⁴ may form a five- or six-membered saturated or unsaturated ring which may contain one or more oxygen, nitrogen, or sulfur atoms together with a carbon atom to which they are attached,
   provided that the compounds of general formula (Ia) wherein X represents CH₂, m is 3, Y represents N, Z represents CH, V represents -CH₂-, and W represents group (i) (wherein R³ and R⁴ represent a hydrogen atom) and wherein X represents CH₂, m is 2, Y represents CH, Z represents N, V represents -CH₂-, and W represents group (i) (wherein R³ and R⁴ represent a hydrogen atom )are excluded.

### DETAILED DESCRIPTION OF THE INVENTION

### Definition

As used herein, the term "lower alkyl" or "lower alkoxy" as a group or a portion of a group means straight-chain or branched alkyl or alkoxy having 1 to 6 carbon atoms, preferably 1 to 4 carbon atoms.

The term "halogen atom" as used herein means a fluorine, chlorine, bromine, or iodine atom.

Examples of lower alkyls include methyl, ethyl, n-propyl, isopropyl, n-butyl, i-butyl, s-butyl, t-butyl, n-pentyl, neopentyl, and n-hexyl. One or more hydrogen atoms of the alkyl may be substituted by a halogen atom. Examples of substituted alkyls include trrluoromethyl, 2-fluoroethyl, difluoroethyl, 2,2,2-trifluoroethyl, trichloromethyl, 2-chloroethyl, dichloroethyl, 1,1,1-trichoroethyl, tribromomethyl, 2-bromoethyl, dibromoethyl, 1,1,1-tribromoethyl, pentafluoroethyl, fluoromethyl, 3,3,3-trifluoropropyl, 4,4,4-trichlorobutyl, 5,5,5-trifluoropentyl, and 6,6,6-trifluorohexyl.

Examples of lower alkoxys include methoxy, ethoxy, n-propoxy, i-propoxy, n-butoxy, i-butoxy, s-butoxy, and t-butoxy. One or more hydrogen atoms of the alkoxy may be substituted. Examples of substituted alkoxys include 2,2,2-trifluoroethoxy, difluoroethoxy, 2,2,2-trichioroethoxy, dichloroethoxy, dibromoethoxy, 2,2,2-tribromoethoxy, fluoromethoxy, difluoromethoxy, trifluoromethoxy, 2-bromoethoxy, 4,4,4-trifluorobutoxy, pentafluoroethoxy, and 3,3, 3-trifluoropropoxy.

### Pharmaceutical composition

Compounds represented by general formula (I) have high binding affinity for dopamine D₄ receptor (see Pharmacological Test Example). It has been confumed that Dopamine D₄ receptors are deeply involved in mental diseases including schizophrenia (Van Tol, et al., Nature, 350, 610 (1991); Seeman.P., et al., Nature, 365, 441 (1993); and Seeman P., Neuropsychopharmacology, 7, 261 (1992)). Therefore, the compounds of general formula (I) are useful as therapeutic agents for mental diseases, anxiety syndrome, and depression.

Thus, according to one aspect of the present invention, there is provided a psychotropic pharmaceutical composition comprising a compound represented by general formula (I) or a pharmaceutically acceptable salt or solvate thereof.

In general formula (I), both R¹ and R² preferably represent a hydrogen atom.

In groups (i) to (iii), R³ and R⁴, which may be the same or different, preferably represent hydrogen, fluorine, chlorine, methyl, methoxy, trifluoromethyl, cyano, i-propyl, or t-butyl.

R³ and R⁴ may form a five- or six-membered saturated or unsaturated ring together with a carbon atom to which they are attached. The saturated or unsaturated ring may contain one or more oxygen, nitrogen, or sulfur atoms. For example, R³ and R⁴ may combine together to form -O-CH₂-O-, -O-CH₂-CH₂-O-, -NH-CH₂-CH₂-, -NHCH₂CH₂CH₂-, -N = CH-CH = CH-, -O-CH₂-CH₂-CH₂-, -SHCH₂CH₂-, or -SHCH₂CH₂CH₂-. Preferably, R³ and R⁴ combine together to form -O-CH₂-O-.

Preferably, substituted group (i) represent 3,4-difluorophenyl, 3,4-methylenedioxyphenyl, 4-chlorophenyl, 2,4-difluorophenyl, 3,4-difluorophenyl, 4-t-butylphenyl, 2-methoxyphenyl, 4-fluorophenyl, 3-fluorophenyl, 4-trifluoromethylphenyl, 4-methoxyphenyl, 4-methylphenyl, 2-methylphenyl, 4-nitrilephenyl, 4-i-propylphenyl, 2-fluorophenyl, 2-chlorophenyl, or 3-methylphenyl.

When n is 0, -(CH₂)ₙ- represents a chemical bond.

Preferred examples of compounds represented by general formula (I) include:
compounds wherein any one of or both Y and Z represent N, V represents -(CH₂)ₙ-, and W represents group (i);
compounds wherein any one of or both Y and Z represent N, V represents -(CH₂)ₙ-, and W represents group (ii);
compounds wherein any one of or both Y and Z represent N, V represents -O-, and W represents group (i); and
compounds wherein any one of or both Y and Z represent N, V represents -(CH₂)ₙ-, and W represents group (iii).

Particularly preferred examples of compounds represented by general formula (I) include:
1-[3-(4-cyclohexanemethyl-1-piperazinyl)-propyl]-2(1H)-quinolinone;
1-[4-(4-cyclohexanemethyl-1-piperazinyl)-butyl]-2(1H)-quinolinone;
1-[3-[4-(3,4-difluorobenzyl)-1-piperazinyl]-propyl]-2(1H)-quinolinone;
1-[4-[4-(3,4-difluorobenzyl)-1-piperazinyl]-butyl]-2(1H)-quinolinone;
1-[3-(4-piperonyl-1-piperazinyl)-propyl]-2(1H)-quinolinone;
1-[4-(4-piperonyl-1-piperazinyl)-butyl]-2(1H)-quinolinone;
1-[3-(4-cyclohexanemethyl-1-piperidinyl)-propyl]-2(1H)-quinolinone;
1-[2-(4-cyclohexanemethyl-1-piperazinyl)-ethyl]-2(1H)-quinolinone;
1-[2-(4-cyclohexanemethyl-1-piperidinyl)-ethyl]-2(1H)-quinolinone,
1-[2-(4-cyclohexanemethyl-1-piperazinyl)-ethyl]-3,4-dihydro-2(1H)-quinolinone;
1-[2-(4-cyclohexanemethyl-1-piperazinyl)-ethyl]-octahydro-2(1H)-quinolinone;
1-[2-(4-cyclohexanemethyl-1-piperidinyl)-ethyl]-octahydro-2(1H)-quinolinone;
1-[3-(4-cyclohexanemethyl-1-piperidinyl)-propyl]-3,4-dihydro-2 (1H)-quinolinone;
1-[3-(4-cyclohexanemethyl-1-piperidinyl)-propyl]-octahydro-2(1H)-quinolinone;
1-[3-[4-(4-chlorobenzyl)-1-piperidinyl]-propyl]-2(1H)-quinolinone,
1-[3-[4-(2,4-difluorobenzyl)-1-piperidinyl]-propyl]-2(1H)-quinolinone;
1-[3-[4-(3,4-difluorobenzyl)-1-piperidinyl]-propyl]-2(1H)-quinolinone;
1-[3-[4-(4-tetrahydropyran)-1-piperidinyl]-propyl]-2(1H)-quinolinone;
1-[3-[4-(4-methoxybenzyl)-1-piperidinyl]-propyl]-2(1H)-quinolinone;
1-[3-[4-(4-tert-butylbenzyl)-1-piperidinyl]-propyl]-2(1H)-quinolinone;
1-[3-[4-phenoxy-1-piperidinyl]-propyl]-2 (1H)-quinolinone;
1-[3-[4-(4-chlorophenoxy)-1-piperidinyl]-propyl]-2(1H)-quinolinone;
1-[4-[4-(benzisoxazole-3-methyl)-1-piperainyl]-propyl]-2(1H)-quinolinone;
1-[3-[4-(2-methoxybenyl)-1-piperazinyl]-propyl]-2(1H)-quinolinone;
1-[3-(1-cyclohexanemethyl-4-piperidinyl)-propyl]-2(1H)-quinolinone;
1-[3-[1-(3,4-difluorobenyl)-4-piperidinyl]-propyl]-2(1H)-quinolinone;
1-[3-[4-(4-chlorophenoxy)-1-piperidinyl]-ethyl]-2(1H)-quinolinone;
1-[3-[1-(3,4-diiluorobenzyl)-4-piperidinyl]-propyl]-4H-benzo[1,4]oxazin-3-one;
1-[3-[1-(4-fluorobenzyl)-4-piperidinyl]-propyl]-4H-benzo[1,4]oxazin-3-one;
1-[3-[1-(3-fluorobenzyl)-4-piperidinyl]-propyl]-4H-benzo[1,4]oxazin-3-one;
1-[3-[1-(4-chlorobenzyl)-4-piperidinyl]-propyl]-4H-benzo[1,4]oxazin-3-one;
1-[3-[1-(3,4-difluorobenzyl)-4-piperidinyl]-propyl]-3,4-dihydro-2 (1H)-quinolinone;
1-[3-[1-(3,4-difluorobenzyl)-4-piperidinyl]-propyl]-4H-benzo[1,4]thiazin-3-one;
1-[3-[1-(4-fluorobenzyl)-4-piperidinyl]-propyl]-4H-benzo[1,4]thiazin-3-one;
1-[3-[1-(4-chlorobenzyl)-4-piperidinyl]-propyl]-4H-benzo[1,4]thiaizin-3-one;
1-[3-[1-(4-trifluoromethylbenzyl)-4-piperidinyl]-propyl]-4H-benzo[1,4]thiazin-3-one;
1-[3-[1-(4-methoxybenzyl)-4-piperidinyl]-propyl]-4H-benzo[1,4]thiazin-3-one;
1-[3-[1-(4-methylbenzyl)-4-piperidinyl]-propyl]-4H-benzo[1,4]thiazin-3-one;
1-[3-[1-(2-methylbenzyl)-4-piperidinyl]-propyl]-4H-benzo[1,4]thiazin-3-one;
1-[3-[1-benzyl-4-piperidinyl]-propyl]-3,4-dihydro-2(1H)-quinolinone;
1-[3-[1-(2,4-difluorobenzyl)-4-piperidinyl]-propyl]-3,4-dihydro-2 (1H)-quinolinone;
1-[3-[1-(4-fluorobenzyl)-4-piperidinyl]-propyl]-3,4-dihydro-2(1H)-quinolinone;
1-[3-[1-(4-chlorobenzyl)-4-piperidinyl]-propyl]-3,4-dihydro-2(1H)-quinolinone;
1-[3-[1-(4-trifluoromethylbenzyl)-4-piperidinyl]-propyl]-3,4-dihydro-2(1H)-quinolinone;
1-[3-[1-(4-methoxybenzyl)-4-piperidlnyl]-propyl]-3,4-dihydro-2(1H)-quinolinone;
1-[3-[1-(2-methylbenzyl)-4-piperidinyl]-propyl]-3,4-dihydro-2(1H)-quinolinone,
1-[3-[1-(4-nitrilebenyl)-4-piperidinyl]-propyl]-4H-benzo[1,4]oxazin-3-one;
1-[3-[1-(4-methoxybenzyl)-4-piperidinyl]-propyl]-4H-benzo[1,4]oxazin-3-one;
1-[3-[1-(4-methylbenzyl)-4-piperidinyl]-propyl]-4H-benzo[1, 4]oxazin-3-one;
1-[3-[1-(4-trifluoromethylbenzyl)-4-piperidinyl)-propyl]-4H-benzo[1,4]oxazin-3-one;
1-[3-[1-(4-isopropylbenzyl)-4-piperidinyl]-propyl]-4H-benzo[1,4]oxazin-3-one;
1-[3-[1-(4-tert-butylbenzyl)-4-piperidinyl]-propyl]-4H-benzo[1,4]oxazin-3-one;
1-[3-[1-(2-fluorobenzyl)-4-piperidinyl]-propyl]-4H-benzo[1,4]oxazin-3-one;
1-[3-[1-(2-chlorobenzyl)-4-piperidlnyi]-propyl]-4H-benzo[1,4]oxazin-3-one;
1-[3-[1-(2-methoxybenzyl)-4-piperidinyl]-propyl]-4H-benzo[1,4]oxazin-3-one;
1-[3-[1-(3-methylbenzyl)-4-piperidinyl]-propyl]-3,4-dihydro-2(1H)-quinolinone; and
1-[3-[1-(4-methylbenzyl)-4-piperidinyl]-propyl]-3,4-dihydro-2(1H)-quinolinone.

The compounds represented by general formula (I) according to the present invention may be formed into pharmaceutically acceptable salts thereof. Such salts include pharmaceutically acceptable non-toxic salts. Preferred salts include alkali metal or alkaline earth metal salts, such as sodium, potassium, and calcium salts, hydrohalogenic acid salts, such as hydrofluoride salts, hydrochloride salts, hydrobromide salts, and hydroiodide salts, inorganic acid salts, such as nitric acid salts, perchloric acid salts, sulfric acid salts, and phosphoric acid salts, lower alkylsulfonic acid salts, such as methanesulfonic acid salts, trifluoromethanesulfonic acid salts, and ethanesulfonic acid salts, arylsulfonic acid salts, such as benzenesulfonic acid salts and p-toluenesulfonic acid salts, organic acid salts, such as fumaric acid salts, succinic acid salts, citric acid salts, tartaric acid salts, oxalic acid salts, maleic acid salts, acetic acid salts, malic acid salts, lactic acid salts, and ascorbic acid salts, and amino acid salts, such as glycine salts, phenylalanine salts, glutamic acid salts, and aspartic acid salts.

The compounds represented by general formula (I) may also be formed into solvate (for example, hydrates) thereof.

The pharmaceutical composition comprising the compound represented by general formula (I) as an active ingredient can be administered to a human and an animal by way of any one of the routes including oral and parenteral administration, such as intravenous injection, intramuscular injection, subcutaneous administration, rectal administration, or percutaneous administration. The pharmaceutical composition comprising the compound according to the present invention as an active ingredient may therefore be formed into an appropriate preparation corresponding to its route.

Specifically, oral preparations includes tablets, capsules, powders, granules, and syrups, and parenteral preparations include injections, such as intravenous injections and intramuscular injections, rectal agents, oleosuppositories and aqueous suppositories.

These preparations may be prepared by conventional methods with commonly used carriers, such as excipients, disintegrators, binders, lubricants, and colorants.

Excipients usable herein includes, for example, lactose, glucose, corn starch, sorbit and crystaine cellulose; disintegrators usable herein include, for example, starch, sodium alginate, gelatin powder, calcium carbonate, calcium citrate and dextrin; binders usable herein include, for example, dimethylcellulose, polyvinyl alcohol, polyvinyl ether, methylcellulose, ethylcellulose, gum arabic, gelatin, hydroxypropylcellulose and polyvinylpyrrolidone; and lubricants usable herein include, for example, talc, magnesium stearate, polyethylene glycol, and hydrogenated vegetable oils.

Furthermore, injections may also be prepared by adding buffers, pH adjustors, or stabilizers, if necessary.

The content of the compound according to the present invention in the pharmaceutical composition depends upon preparations and is generally in the range from 0.1 to 50% by weight, preferably from about 0.5 to 20% by weight, based on the whole composition.

The dosage is appropriately deterntined individually in view of the age, body weight and sex of patients, diseases and the seriousness of symptoms, and ranges generally from 1 to 1,000 mg/adult/day, preferably from 1 to 300 mg/adult/day, which is administered once or in several portions a day.

The terms "psychotropic agent" and "psychotropic pharmaceutical composition" as used herein refer to therapeutic agents for diseases based on functional disturbance in a dopamine nervous system, including antipsychotic agents, antianxiety agents, and antidepressants. In addition, the terms "therapy" and "treatment" may be used in the meaning of "prevention" or "prophylaxis".

According to the present invention, there is provided a method for treatment of mental diseases (for example, schizophrenia and mania), anxiety disorders, and/or depression, including the step of administering to a human or an animal suffering from such diseases, an effective amount of the compound represented by general formula (I) or a pharmaceutically acceptable salt or solvate. The compound represented by general formula (I) may be administered in the same manner as described above.

### Compound represented by general formula (Ia)

According to a second aspect of the present invention, there is provided a novel compound represented by general formula (Ia).

Examples of preferred compounds represented by general formula (Ia) are the same as those described above in connection with the compounds represented by general formula (I).

Examples of preferred salts and solvates of the compounds represented by general formula (Ia) are the same as those described above in connection with the compounds represented by general formula (I).

In the definition of general formula (Ia), the compounds wherein X represents CH₂, m is 3, Y represents N, Z represents CH, V represents -CH₂-, and W represents group (i) (R³ and R⁴ represent a hydrogen atom) and wherein X represents CH₂, m is 2, Y represents CH, Z represents N, V represents -CH₂-, and W represents group (i) (R³ and R⁴ represent a hydrogen atom) are excluded from those represented by general formula (I).

### Production of compounds

The compound represented by general formula (I) may be produced by the following processes.

### Process 1

The compound represented by general formula (I) according to the present invention may be produced by reacting a compound represented by general formula (II): 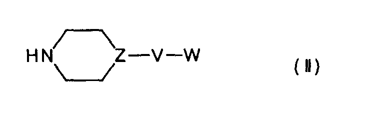 wherein Z, V, and W are as defined in general formula (I), with a compound represented by general formula (III): 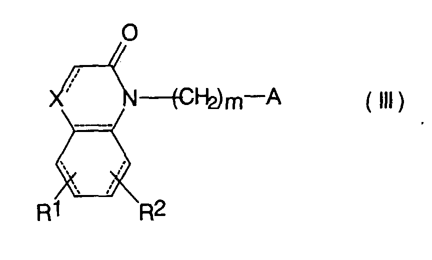 wherein a solid line with a dotted line represents a single bond or a double bond, m is an integer of 1 to 4, R¹, R², and X are as defined in general formula (I), and A represents a halogen atom or a leaving group (for example, p-toluenesulfonyloxy or methanesulfonyloxy), in a non-reactive solvent (for example, anhydrous acetonitrile, dimethylformamide, or tetrahydrofuran) in the presence of an acid scavenger and optionally a miner amount of potassium iodide at a temperature of 20 to 110°C, preferably 40 to 80°C, for 2 to 24 hr, generally 2 to 8 hr.

Acid scavengers usable in the reaction include, for example, alkali carbonates, such as potassium carbonate and sodium bicarbonate, or organic amines, such as triethylamine.

Alternatively, the compound represented by general formula (I) according to the present invention may be produced by reacting the compound represented by formula (II) with a compound represented by general formula (IV): 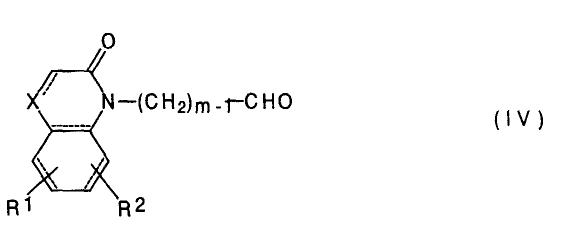 wherein a solid line with a dotted line represents a single bond or a double bond, m is an integer of 1 to 4, and R¹, R², and X are as defined above in connection with general formula (I), in a non-reactive solvent (for example, dichloroethane, tetrahydrofuran, or dimethylsulfoxide) in the presence of NaBH(OAc)₃ (wherein Ac represents acetyl), NaBH₃CN, or AcOH at a temperature of 20 to 50°C, preferably 30°C, for 2 to 48 hr, generally 5 hr.

### Process 2

The compound represented by general formula (I) according to the present invention may be produced by reacting a compound represented by general formula (V): 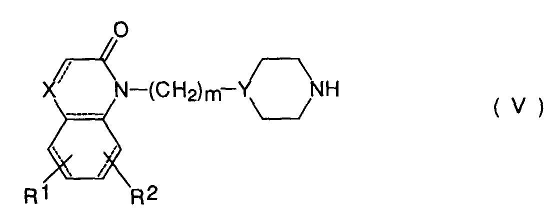 wherein a solid line with a dotted line represents a single bond or a double bond, m is an integer of 1 to 4, R¹, R², X, and Y are as defined in general formula (I), with a compound represented by general formula (VI) or (VII): 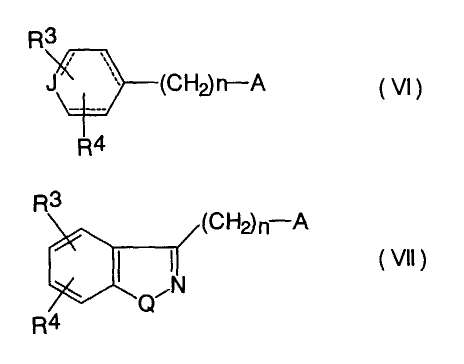 wherein n is an integer of 1 to 4, R³, R⁴, J, and Q are as defined in general formula (I), A represents a halogen atom or a leaving group (for example, p-toluenesulfonyloxy or methanesulfonyloxy), in a non-reactive solvent (for example, anhydrous acetonitrile, dimethylformamide, or tetrahydrofuran) in the presence of an acid scavenger and optionally a miner amount of potassium iodide at a temperature of 20 to 110°C, preferably 40 to 80°C, for 2 to 24hr, generally 2 to 8 hr.

Acid scavengers usable in the reaction include, for example, alkali carbonates, such as potassium carbonate and sodium bicarbonate, or organic amines, such as triethylamine.

Alternatively, the compound represented by general formula (I) according to the present invention may be produced by reacting a compound represented by general formula (V) with a compound represented by general formula (VIII) or (IX): 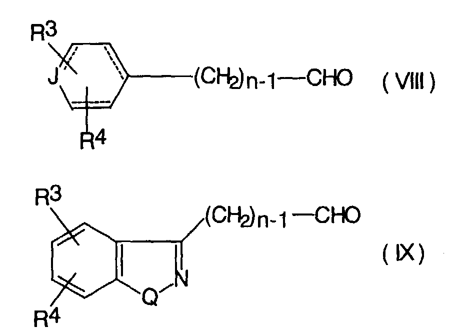 wherein n is an integer of 1 to 4, R³, R⁴, and Q are as defined in general formula (I),
in a non-reactive solvent (for example, dichloroethane, tetrahydrofuran, or dimethylsulfoxide) in the presence of NaBH (OAc)₃, NaBH₃CN, or AcOH at a temperature of 20 to 50°C, preferably 30°C, for 2 to 48 hr, generally 5 hr.

### EXAMPLES

The present invention is further illustrated by the following examples and test examples that are not intended as a limitation of the invention.

### Preparation Example 1 1-(3-Bromopropyl)-2(1H)-quinolinone

2-Hydroxyquinoline (1.5 g) was dissolved in dimethylformamide (30 ml). 64% sodium hydride (428 mg) was added to the solution. The mixture was stirred at 60°C for 30 min and then cooled to a room temperature. 1,3-Dibromopropane (10.4 g) was added thereto, and the mixture was stirred at a room temperature for 4 hr. The reaction solution was evaporated under the reduced pressure. Dichloromethane was added to the residue. The dichloromethane solution was washed with water and dried over anhydrous magnesium sulfate. The solvent was evaporated under the reduced pressure to give an oil. The oil was purified by column chromatography on silica gel to give 1-(3-bromopropyl)-2(1H)-quinolinone (1.39 g).

### Example 1 1-[3-(4-Cyclohexanemethyl-1-piperazinyl)-propyl]-2(1H)-quinolinone

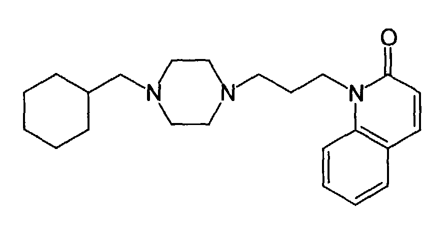

1-(3-Bromopropyl)-2(1H)-quinolinone (250 mg) obtained in Preparation example 1, cyclohexanemethylpiperazine (171 mg), and potassium carbonate (195 mg) were suspended in dimethylformamide (3 ml). The suspension was heated at 80°C for 10 hr. Dichloromethane was added to the reaction solution. The mixture was washed with water and dried over anhydrous magnesium sulfate. The solvent was evaporated under the reduced pressure to give an oil. The oil was purified by column chromatography on silica gel to give the title compound (272 mg).
¹H NMR (CDCl₃) δ (ppm): 0.80 - 0.98 (2H, m), 1.09 - 1.30 (3H, m), 1.42 - 1.55 (1H, m), 1.61 - 1.80 (5H, m), 1.93 (2H, tt, J = 7.7 and 7.2 Hz), 2.13 (2H, d, J = 7.2 Hz), 2.36 - 2.58 (8H, m), 2.48 (2H, t, J = 7.2 Hz), 4.35 (2H, t, J = 7.7 Hz), 6.69 (1H, d, J = 9.5 Hz), 7.18- 7.24 (1H, m), 7.50 - 7.58 (3H, m), 7.66 (1H, d, J = 9.5 Hz).
Mass spectrum EIMS, m/z 367 (M⁺).

### Preparation Example 2 1-(4-Bromobutyl)-2(1H)-quinolinone

1-(4-Bromobutyl)-2(1H)-quinolinone was syhthesized in the same manner as in Preparation Example 1, except that 1,4-dibromobutane was used instead of 1,3-dibromopropane.

### Example 2 1-[4-(4-Cyclohexanemethyl-1-piperazinyl)-butyl]-2(1H)-quinolinone

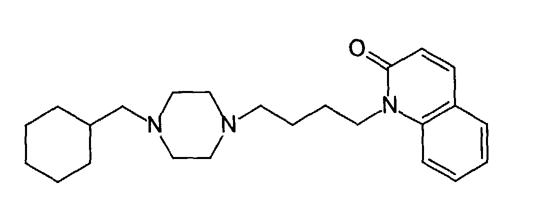

The title compound was syhthesized in the same manner as in Example 1, except that 1-(4-bromobutyl)-2(1H)-quinolinone obtained in preparation Example 2 was used instead of 1-(3-bromopropyl)-2(1H)-quinolinone.
¹N NMR (CDCl₃) δ (ppm): 0.80-0.94 (2H, m), 1.09-1.30 (3H, m), 1.42-1.55 (1H, m), 1.62-1.84 (9H, m), 2.14 (2H, d, J = 6.9 Hz), 2.30-2.70 (10H, m), 4.32 (2H, t, J = 7.4 Hz), 6.69 (1H, d, J = 9.5 Hz), 7.20-7.25 (1H, m), 7.48 (1H, d, J = 9.0 Hz), 7.53-7.59 (2H, m), 7.66 (1H, d, J = 9.5 Hz).
Mass spectrum EIMS, m/z 381 (M⁺).

### Example 3 1-[3-[4-(3,4-Difluorobenzyl)-1-piperazinyl]-propyl]-2(1H)-quinolinone

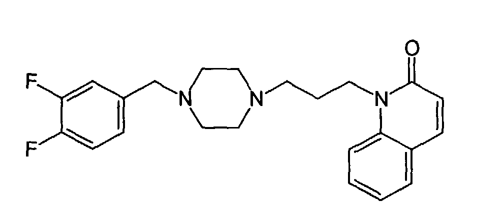

The title compound was synthesized in the same manner as in Example 1, except that 3,4-difluorobenzyl-1-piperazine was used instead of cyclohexanemethylpiperarine.
¹H NMR (CDCl₃) δ (ppm): 1.93 (2H, tt, J = 7.4 and 6.9 Hz), 2.40-2.58 (8H, m), 2.49 (2H, t, J = 6.9 Hz), 3.45 (2H, s), 4.35 (2H, t, J = 7.4 Hz), 6.69 (1H, d, J = 9.5 Hz), 6.99-7.12 (2H, m), 7.14-7.20 (1H, m), 7.19-7.24 (1H, m), 7.47-7.58 (3H, m), 7.66 (1H, d, J = 9.5 Hz).
Mass spectrum EIMS, m/z 397 (M⁺).

### Example 4 1-[4-[4-(3,4-Difluorobenzyl)-1-piperazinyl]-butyl]-2(1H)-quinolinone

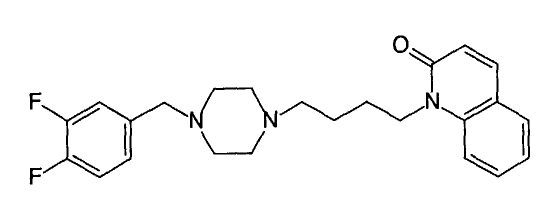

The title compound was syhthesized in the same manner as in Example 2, except that 3, 4-difluorobenzyl-1-piperazine was used instead of cyclohexanemethylpiperazine.
¹H NMR (CDCl₃) δ (ppm): 1.58-1.82 (4H, m), 2.37-2.57 (8H, m), 2.43 (2H, t, J = 7.2 Hz), 3.45 (2H, s), 4.32 (2H, t, J = 7.6 Hz), 6.69 (1H, d, J = 9.5 Hz), 6.98-7.13 (2H, m), 7.13-7.26 (2H, m), 7.46 (1H, d, J = 8.5 Hz), 7.50-7.58 (2H, m), 7.66 (1H, d, J = 9.5 Hz).
Mass spectrum EIMS, m/z 411 (M⁺).

### Example 5 1-[3-(4-Piperonyl-1-piperazinyl)-propyl]-2(1H)-quinolinone

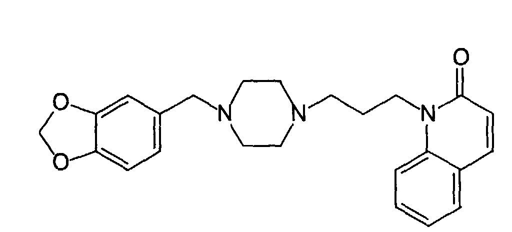

The title compound was synthesised in the same manner as in Example 1, except that 1-piperonylpiperazine was used instead of cyclohexanemethylpiperazine.
¹N NMR (CDCl₃) δ (ppm): 1.93 (2H, tt, J = 7.4 and 6.9 Hz), 2.38-2.60 (8H, m), 2.48 (2H, t, J = 6.9 Hz), 3.42 (2H, s), 4.35 (2H, t, J = 7.4 Hz), 5.94 (2H, s), 6.69 (1H, d, J = 9.5 Hz), 6.74 (1H, s), 6.75 (1H, s), 6.85 (1H, s), 7.18-7.24 (1H, m), 7.48-7.58 (3H, m), 7.66 (1H, d, J = 9.5 Hz).
Mass spectrum EIMS, m/z 405 (M⁺).

### Example 6 1-[4-(4-Piperonyl-1-piperazinyl)-butyl]-2(1H)-quinolinone

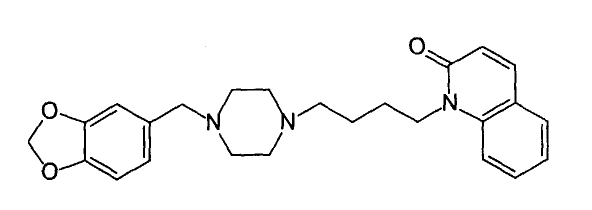

The title compound was synthesised in the same manner as in Example 2, except that 1-piperonylpiperazine was used instead of cyclohexanemethylpiperazine.
¹H NMR (CDCl₃) δ (ppm): 1.60-1.83 (4H, m), 2.34-2.61 (8H, m), 2.43 (2H, t, J = 7.2 Hz), 3.43 (2H, s), 4.31 (2H, t, J = 7.4 Hz), 5.94 (2H, s), 6.69 (1H, d, J = 9.5 Hz), 6.74 (1H, s), 6.74 (1H, s), 6.85 (1H, s), 7.19-7.24 (1H, m), 7.45-7.58 (3H, m), 7.65 (1H, d, J = 9.5 Hz).
Mass spectrum EIMS, m/z 419 (M⁺).

### Example 7 1-[3-(4-Cyclohexanemethyl-1-piperidinyl)-propyl]-2(1H)-quinolinone

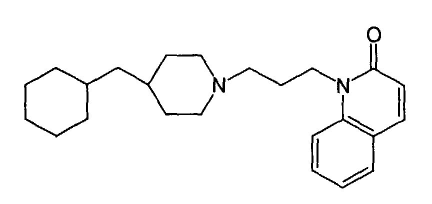

The title compound was synthesised in the same manner as in Example 1, except that cyclohexanemethylpiperidine was used instead of cyclohexanemethylpiperazine.
¹H NMR (CDCl₃) δ (ppm): 0.77-0.91 (2H, m), 1.09 (2H, t, J = 7.2 Hz), 1.13-1.30 (7H, m), 1.26-1.40 (1H, m), 1.59-1.74 (6H, m), 1.87-2.00 (4H, m), 2.45 (2H, t, J= 7.5 Hz), 2.92 (2H, brd, J = 11.3 Hz), 4.35 (2H, t, J = 7.5 Hz), 6.69 (1H, d, J = 9.4 Hz), 7.18-7.24 (1H, m), 7.50-7.58 (3H, m), 7.66 (1H, d, J = 9.4 Hz).
Mass spectrum EIMS, m/z 366 (M⁺).

### Preparation Example 3 1-(2-Propenyl)-2(1H)-quinolinone

2-Hydroxyquinoline (100 mg) was dissolved in dimethylformamide (2 ml). 64% sodium hydride (29 mg) was added to the solution. The mixture was stared at 60°C for 30 min and cooled to a room temperature. Allyl bromide (83 mg) was added thereto, and the mixture was stirred at a room temperature for 10 hr. The reaction solution was evaporated under the reduced pressure. Dichloromethane was added to the residue. The mixture was washed with water. The organic layer was dried over anhydrous magnesium sulfate. The solvent was evaporated under the reduced pressure to give an oil. The oil was purified by column chromatography on silica gel to give 1-(2-propenyl)-2(1H)-quinolinone (81 mg).

### Preparation Example 4 1-Formylmethyl-2(1H)-quinolinone

1-(2-Propenyl)-2(1H)-quinolinone (590 mg) obtained in Preparation Example 3 was dissolved in dioxane (20 ml). Osmium tetraoxide (81 mg) was added to the solution. The mixture was stirred at a room temperature for 15 min under light shield conditions. Water (7 ml) was added thereto, and a solution of sodium periodate (1.36 g) in water (9 ml) was further added thereto over a period of 20 min. The mixture was stirred at a a room temperature for 1.5 hr. Further, sodium periodate (1 g) was added thereto by portions. The mixture was stirred for 10 hr. A saturated aqueous sodium thiosulfate solution was added to the reaction solution. The mixture was extracted with dichloromethane. The organic layer was washed with saturated saline and dried over anhydrous magnesium sulfate. The solvent was evaporated under the reduced pressure to give an oil. The oil was purified by column chromatography on silica gel to give 1-formylmethyl-2(1H)-quinolinone (450 mg).

### Example 8 1-[2-(4-Cyclohexanemethyl-1-piperazinyl)-ethyl]-2(1H)-quinolinone

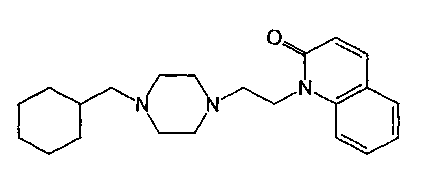

Cyclohexanemethylpiperazine (170 mg) was dissolved in 1,2-dichloroethane (3 ml). 1-Formylmethyl-2(1H)-quinolinone (225 mg) obtained in Preparation Example 4, sodium triacetoxyborohydride (296 mg), and acetic acid (0.05 ml) were added in that order to the solution. The mixture was stirred at a room temperature for 15 hr. The reaction solution was evaporated under the reduced pressure. Dichloromethane was added to the residue. The mixture was washed with a saturated aqueous sodium hydrogencarbonate solution. The organic layer was dried over anhydrous magnesium sulfate. The solvent was evaporated under the reduced pressure to give an oil. The oil was purified by column chromatography on silica gel to give the title compound (331 mg).
¹N NMR (CDCl₃) δ (ppm): 0.80 - 0.95 (2H, m), 1.09 - 1.29 (3H, m), 1.42 - 1.54 (1H, m), 1.60 - 1.82 (5H, m), 2.13 (2H, d, J = 7.2 Hz), 2.37 - 2.56 (4H, m), 2.58 - 2.74 (4H, m), 2.68 (2H, t, J = 7.8 Hz), 4.46 (2H, t, J = 7.8 Hz), 6.69 (1H, d, J = 9.4 Hz), 7.18 - 7.25 (1H, m), 7.41 - 7.46 (1H, m), 7.52 - 7.60 (2H, m), 7.66 (1H, d, J = 9.4 Hz).
Mass spectrum FABMS, m/z 354 (M+1).

### Example 9 1-[2-(4-Cyclohexanemethyl-1-piperidinyl)-ethyl]-2(1H)-quinolinone

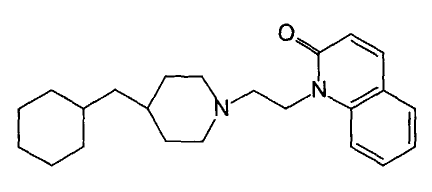

The title compound was synthesised in the same manner as in Example 8, except that cyclohexanemethylpiperidine was used instead of cyclohexanemethylpiperazine.
¹H NMR (CDCl₃) δ (ppm): 0.78 - 0.91 (2H, m), 1.10 (2H, t, J = 6.9 Hz), 1.14 - 1.30 (7H, m), 1.26 - 1.43 (1H, m), 1.59 - 1.74 (6H, m), 2.11 (2H, dt, J = 11.5 and 3.3 Hz), 2.64 (2H, t, J = 8.0 Hz), 3.05 (2H, brd, J = 11.5 Hz), 4.46 (2H, t, J = 8.0 Hz), 6.69 (1H, d, J = 9.5 Hz), 7.19 - 7.25 (1H, m), 7.45 (1H, d, J = 9.0 Hz), 7.52 - 7.58 (2H, m), 7.66 (1H, d, J = 9.5 Hz).
Mass spectrum FABMS, m/z 353 (M+1).

### Example 10

### (A)1-[2-(4-Cyclohexanemethyl-1-piperazinyl)-ethyl]-3,4-dihydro-2(1H)-quinolinone

### (B)1-[2-(4-Cyclohexanemethyl-1-piperazinyl)-ethyl]-octahydro-2(1H)-quinolinone

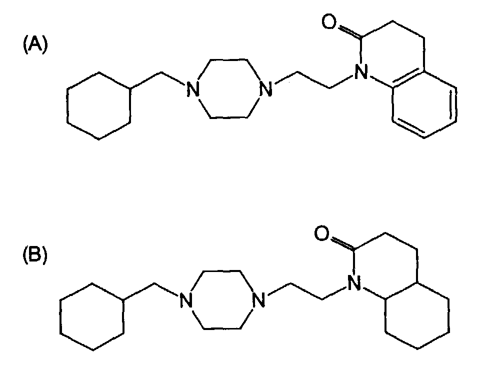

1-[2-(4-Cyclohexanemethyl-1-piperazinyl)-ethyl]-2(1H)-quinolinone (160 mg) obtained in Example 8 was dissolved in acetic acid (8 ml). 95% platinum oxide (6 mg) was added to the solution. The mixture was stirred in a hydrogen atmosphere at a a room temperature for two days. 95% platinum oxide (32 mg) was further added thereto, and the mixture was stirred for 3 days. The insolubles were removed by filtration. The filtrate was evaporated under the reduced pressure. Dichloromethane was added to the residue. The mixture was washed with a saturated aqueous sodium hydrogencarbonate solution and dried over anhydrous magnesium sulfate. The solvent was evaporated under the reduced pressure to give an oil. The oil was purified by column chromatography on silica gel to give title compound (A) (63 mg) and title compound (B) (69 mg).
(A): ¹H NMR (CDCl₃) δ (ppm): 0.80 - 0.93 (2H, m), 1.08 - 1.30 (3H, m), 1.40 - 1.53 (1H, m), 1.60 - 1.80 (8H, m), 2.12 (2H, d, J = 6.9 Hz), 2.35 - 2.50 (4H, m), 2.51 - 2.68 (4H, m), 2.89 (2H, t, J = 7.7 Hz), 4.08 (2H, t, J = 7.7 Hz), 7.00 (1H, t, J = 7.4 Hz), 7.07 (1H, t, J = 8.0 Hz), 7.15 (1H, d, J = 7.4 Hz), 7.23 (1H, dd, J = 8.0 and 7.4 Hz).
Mass spectrum FABMS, m/z 356 (M+1).
(B): ¹H NMR (CDCl₃) δ (ppm): 0.79 - 0.93 (2H, m), 1.08 - 1.28 (4H, m), 1.28 - 1.53 (5H, m), 1.53 - 1.81 (10H, m), 1.86 - 1.96 (1H, m), 1.98 - 2.10 (2H, m), 2.10 (2H, d, J = 6.9 Hz), 2.28 - 2.62 (10H, m), 2.92 - 3.06 (1H, m), 3.27 (1H, dt, J = 11.8 and 4.1 Hz), 3.82 - 3.94 (1H, m).
Mass spectrum FABMS, m/z 362 (M+1).

### Example 11 1-[2-(4-Cydohexanemethyl-1-piperidinyl)-ethyl]-octahydro-2(1H)-quinolinone

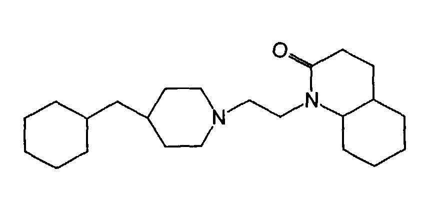

1-[2-(4-Cyclohexanemethyl-1-piperidinyl)-ethyl]-2(1H)-qulnolinone (64 mg) obtained in Example 9 was dissolved in acetic acid (3 ml). 95% platinum oxide (22 mg) was added to the solution. The mixture was stirred in a hydrogen atmosphere at a a room temperature for 15 hr. The insolubles were removed by filtration. The filtrate was evaporated under the reduced pressure. Dichloromethane was added to the residue. The mixture was washed with a saturated aqueous sodium hydrogencarbonate solution. The organic layer was dried over anhydrous magnesium sulfate. The solvent was evaporated under the reduced pressure to give an oil. The oil was purified by column chromatography on silica gel to give the title compound (45 mg).
¹H NMR (CDCl₃) δ (ppm): 0.77 - 0.92 (2H, m), 1.03 - 1.10 (2H, t, J = 6.9 Hz), 1.10 - 1.53 (12H, m), 1.53 - 1.82 (11H, m), 1.96 - 2.14 (4H, m), 2.27 - 2.57 (4H, m), 2.79 - 3.07 (10H, m), 3.26 (1H, dt, J = 11.7 and 3.9 Hz), 3.81 - 3.94 (1H, m).
Mass spectrum FABMS, m/z 361 (M+1).

### (A)1-[3-(4-Cyclohexanemethyl-1-piperidinyl)-propyl]-3,4-dihydro-2(1H)-quinolinone

### (B)1-[3-(4-Cyclohexanemethyl-1-piperidinyl)-propyl]-octahydro-2(1H)-quinolinone

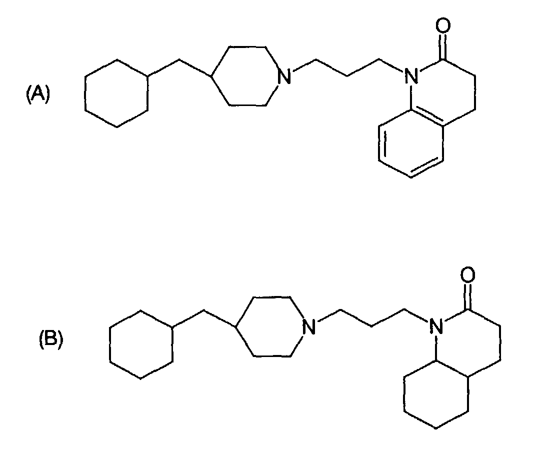

1-[3-(4-Cyclohexanemethyl-1-piperidinyl)-propyl]-2(1H)-quinolinone (250 mg) obtained in Example 7 was dissolved in acetic acid (12 ml). 95% platinum oxide (10 mg) was added to the solution. The mixture was stared in a hydrogen atmosphere at a room temperature for 24 hr. 95% platinum oxide (50 mg) was further added thereto. The mixture was stirred for 24 hr. The insolubles were removed by filtration. The filtrate was evaporated under the reduced pressure. Dichloromethane was added to the residue. The mixture was washed with a saturated aqueous sodium hydrogencarbonate solution. The organic layer was dried over anhydrous magnesium sulfate. The solvent was evaporated under the reduced pressure to give an oil. The oil was purified by column chromatography on silicagel to give title compound (A) (29 mg) and (B) (55 mg).
(A): ¹H NMR (CDCl₃) δ (ppm): 0.76 - 0.91 (2H, m), 1.05 - 1.12 (2H, t, J = 7.2 Hz), 1.12 - 1.29 (6H, m), 1.24 - 1.40 (1H, m), 1.57 - 1.76 (7H, m), 1.79 - 1.96 (4H, m), 2.39 (2H, t, J = 7.4 Hz), 2.59 - 2.67 (2H, m), 2.88 (4H, m), 3.97 (2H, t, J = 7.4 Hz), 6.99 (1H, dd, J = 7.2 and 1.0 Hz), 7.09 (1H, d, J = 8.0 Hz), 7.15 (1H, dd, J = 7.2 and 1.5 Hz), 7.23 (1H, ddd, J = 8.0, 7.2 and 1.5 Hz).
Mass spectrum FABMS, m/z 369 (M+1).
(B): ¹H NMR (CDCl₃) δ (ppm): 0.75 - 0.92 (2H, m), 1.04 - 1.10 (2H, m), 1.10 - 1.54 (12H, m), 1.54 - 1.80 (12H, m), 1.80 - 1.95 (3H, m), 1.98 - 2.13 (2H, m), 2.22 - 2.50 (4H, m), 2.78 - 2.95 (3H, m), 3.27 (1H, dt, J = 11.2 and 3.6 Hz), 3.73 - 3.87 (1H, m).
Mass spectrum FABMS, m/z 375 (M+1).

### Example 13 1-[3-[4-(4-Chlorobenzyl)-1-piperidinyl]-propyl]-2(1H)-quinolinone

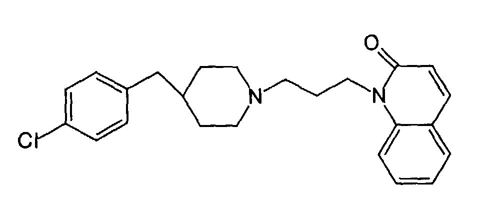

The title compound was synthesized in the same manner as in Example 1, except that 4-(4-chlorobenzyl)-1-piperidine was used instead of cyclohexanemethylpiperazine.
¹H NMR (CDCl₃) δ (ppm): 1.20 - 1.40 (2H, m), 1.41 - 1.58 (1H, m), 1.58 - 1.69 (2H, m), 1.82 - 2.01 (4H, m), 2.45 (2H, t, J = 7.1 Hz), 2.51 (2H, m), 2.91 (2H, brd, J = 11.6 Hz), 4.34 (2H, t, J = 7.5 Hz), 6.69 (1H, d, J = 9.5 Hz), 7.07 (2H, d, J = 8.5 Hz), 7.18 - 7.25 (1H, m), 7.24 (2H, d, J = 8.5 Hz), 7.47 - 7.59 (3H, m), 7.66 (1H, d, J = 9.5 Hz).

| High Mass (FAB, M+1): | |
|---|---|
| Calculated C₂₄H₂₈N₂OCl: | 395.1890. |
| Found: | 395.1901. |

### Example 14 1-[3-[4-(2,4-Difluorobenzyl)-1-piperidinyl]-propyl]-2(1H)-quinolinone

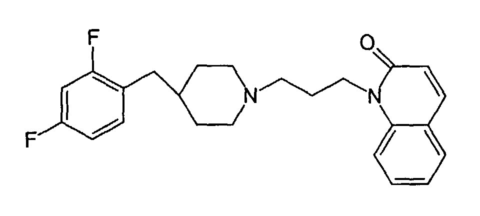

The title compound was synthesised in the same manner as in Example 1, except that 4-(2,4-difluorobenzyl)-1-piperidine was used instead of cyclohexanemethylpiperazine.
¹H NMR (CDCl₃) δ (ppm): 1.26 - 1.40 (2H, m), 1.46 - 1.60 (1H, m), 1.63 (2H, brd, J = 12.8 Hz), 1.84 - 2.00 (4H, m), 2.45 (2H, t, J = 7.1 Hz), 2.54 (2H, d, J = 6.9 Hz), 2.92 (2H, brd, J = 11.5 Hz), 4.34 (2H, t, J = 7.4 Hz), 6.69 (1H, d, J = 9.5 Hz), 6.73 - 6.82 (2H, m), 7.09 (1H, dt, J = 6.7 and 9.0 Hz), 7.22 (1H, ddd, J = 8.2, 7.4 and 1.5 Hz), 7.48 - 7.58 (3H, m), 7.66 (1H, d, J = 9.5 Hz).

| High Mass (FAB, M+1): | |
|---|---|
| Calculated C₂₄H₂₇N₂OF₂: | 397.2091. |
| Found: | 397.2038 |

### Example 15 1-[3-[4-(3,4-Difluorobenxyl)-1-piperidinyl]-propyl]-2(1H)-quinolinone

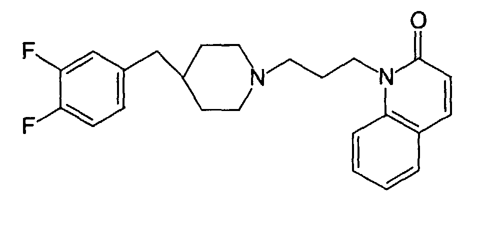

The title compound was synthesized in the same manner as in Example 1, except that 4-(3,4-difluorobenzyl)-1-piperidine was used instead of cyclohexanemethylpiperazine.
¹H NMR (CDCl₃) δ (ppm): 1.23 - 1.36 (2H, m), 1.50 (1H, tt, J = 7.4 and 3.6 Hz), 1.58 - 1.72 (2H, m), 1.86 - 1.98 (4H, m), 2.46 (2H, t, J = 7.4 Hz), 2.50 (2H, d, J = 7.2 Hz), 2.93 (2H, brd, J = 11.5 Hz), 4.34 (2H, t, J = 7.4 Hz), 6.69 (1H, d, J = 9.5 Hz), 6.81 - 6.86 (1H, m), 6.92 (1H, ddd, J = 11.3, 7.7 and 2.1 Hz), 7.05 (1H, dt, J = 10.3 and 8.5 Hz), 7.19 - 7.25 (1H, m), 7.49 (1H, d, J = 8.0 Hz), 7.53 (1H, dd, J = 6.9 and 1.5 Hz), 7.55 (1H, d, J = 8.0 Hz), 7.67 (1H, d, J = 9.5 Hz).

| High Mass (FAB, M+1): | |
|---|---|
| Calculated C₂₄H₂₇N₂OF₂: | 397.2091. |
| Found: | 397.2095. |

### Example 16 1-[3-[4-(4-tetrahydropyran)-1-piperidinyl]-propyl]-2(1H)-quinolinone

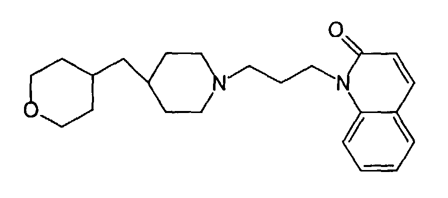

The title compound was synthesized in the same manner as in Example 1, except that 4-(4-tetrahydropyran)-1-piperidine was used instead of cyclohexanemethylpiperazine.
¹H NMR (CDCl₃) δ (ppm): 1.12 - 1.46 (8H, m), 1.51 - 1.73 (4H, m), 1.86 - 2.02 (4H, m), 2.47 (2H, t, J = 7.1 Hz), 2.93 (2H, brd, J = 11.8 Hz), 3.37 (2H, dt, J = 1.7 and 10.6 Hz), 3.94 (2H, dd, J = 11.3 and 3.6 Hz), 4.35 (2H, t, J = 7.5 Hz), 6.69 (1H, d, J = 9.5 Hz), 7.18 - 7.25 (1H, m), 7.48 - 7.59 (3H, m), 7.67 (1H, d, J = 9.5 Hz).

| High Mass (FAB, M+1): | |
|---|---|
| Calculated C₂₃H₃₃N₂ O₂: | 369.2542. |
| Found: | 369.2556. |

### Example 17 1-[3-[4-(4-Methoxybenzyl)-1-piperidinyl]-propyl]-2 (1H)-quinolinone

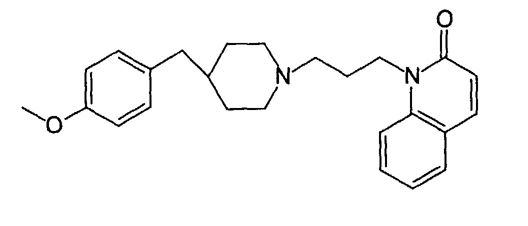

The title compound was synthesized in the same manner as in Example 1, except that 4-(4-methoxybenzyl)-1-piperidine was used instead of cyclohexemethylpiperazine.
¹H NMR (CDCl₃) δ (ppm): 1.40 - 1.60 (3H, m), 1.70 (2H, brd, J = 11.7 Hz), 2.00 - 2.16 (4H, m), 2.50 (2H, d, J = 6.4 Hz), 2.57 - 2.68 (2H, m), 3.02 - 3.12 (2H, m), 3.79 (3H, s), 4.35 (2H, t, J = 7.5 Hz), 6.68 (1H, d, J = 9.4 Hz), 6.82 (2H, d, J = 8.6 Hz), 7.05 (2H, d, J = 8.6 Hz), 7.23 (1H, ddd, J = 8.1, 7.5 and 0.8 Hz), 7.48 - 7.60 (3H, m), 7.67 (1H, d, J = 9.4 Hz).

| High Mass (FAB, M+1): | |
|---|---|
| Calculated C₂₅H₃₁N₂ O₂: | 391.2386. |
| Found: | 391.2372. |

### Example 18 1-[3-[4-(4-tert.-Butylbenzyl)-1-piperidinyl]-propyl]-2 (1H)-quinolinone

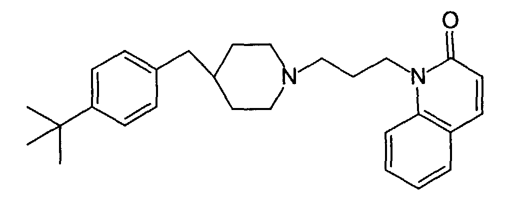

The title compound was synthesized in the same manner as in Example 1, except that 4-(4-tert-butylbenzyl)-1-piperidine was used instead of cyclohexanemethylpiperazine.
¹H NMR (CDCl₃) δ (ppm): 1.24 - 1.38 (2H, m), 1.31 (9H, s), 1.48 - 1.60 (1H, m), 1.67 (2H, brd, J = 13.0 Hz), 1.86 - 1.98 (4H, m), 2.45 (2H, t, J = 7.2 Hz), 2.51 (2H, d, J = 6.9 Hz), 2.92 (2H, brd, J = 11.7 Hz), 4.34 (2H, t, J = 7.5 Hz), 6.68 (1H, d, J = 9.4 Hz), 7.08 (2H, d, J = 8.3 Hz), 7.19 - 7.24 (1H, m), 7.30 (2H, d, J = 8.3 Hz), 7.50 - 7.58 (3H, m), 7.65 (1H, d, J = 9.4 Hz).

| High Mass (FAB, M+1): | |
|---|---|
| Calculated C₂₈H₃₇N₂O: | 417.2906. |
| Found: | 417.2876. |

### Example 19 1-[3-[4-Phenoxy-1-piperidinyl]-propyl]-2(1H)-quinolinone

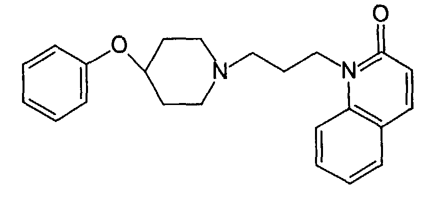

The title compound was synthesized in the same manner as in Example 1, except that 4-phenoxy-1-piperidine was used instead of cyclohexanemethylpiperazine.
¹H NMR (CDCl₃) δ (ppm): 1.78 - 1.90 (2H, m), 1.96 (2H, dt, J = 14.7 and 7.2 Hz), 2.00 - 2.08 (2H, m), 2.26 - 2.37 (2H, m), 2.52 (2H, t, J = 6.9 Hz), 2.80 (2H, brs), 4.33 (1H, tt, J = 7.7 and 3.7 Hz), 4.38 (2H, t, J = 7.5 Hz), 6.70 (1H, d, J = 9.4 Hz), 6.89 - 6.96 (3H, m), 7.20 - 7.31 (3H, m), 7.51 - 7.58 (3H, m), 7.68 (1H, d, J = 9.4 Hz).

| High Mass (FAB, M+1): | |
|---|---|
| Calculated C₂₃H₂₇N₂ O₂: | 363.2072. |
| Found: | 363.2043. |

### Example 20 1-[3-[4-(4-Chlorophenoxy)-1-piperidinyl]-propyl]-2(1H)-quinolinone

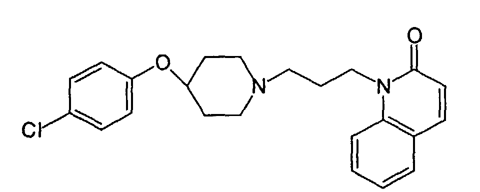

The title compound was synthesized in the same manner as in Example 1, except that 4-chlorophenoxy-1-piperidine was used instead of cyclohexanemethylpiperazine.
¹H NMR (CDCl₃) δ (ppm): 1.77 - 1.88 (2H, m), 1.91 - 2.06 (4H, m), 2.26 - 2.36 (2H, m), 2.51 (2H, t, J = 6.9 Hz), 2.79 (2H, brs), 4.28 (1H, tt, J = 7.7 and 3.7 Hz), 4.37 (2H, t, J = 7.5 Hz), 6.70 (1H, d, J = 9.4 Hz), 6.84 (2H, d, J = 9.2 Hz), 7.20 - 7.23 (1H, s), 7.22 (2H, d, J = 9.2 Hz), 7.50 - 7.59 (3H, m)7.68 (1H, d, J = 9.4 Hz).

| High Mass (FAB, M+1): | |
|---|---|
| Calculated C₂₃H₂₆N₂O₂Cl: | 397.1682. |
| Found: | 397.1675. |

### Example 21 1-[4-[4-(Benzisoxazole-3-methyl)-1-piperazinyl]-propyl]-2 (1H)-quinolinone

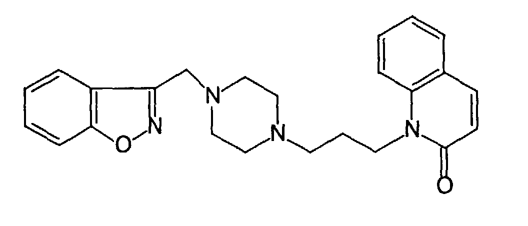

The title compound was synthesized in the same manner as in Example 1, except that 4- (benzisoxazole-3-methyl)-1-piperidine was used instead of cyclohexanemethylpiperazine.
¹H NMR (CDCl₃) δ (ppm): 1.93 (2H, tt, J = 7.6 and 7.1 Hz), 2.46 - 2.66 (8H, m), 2.49 (2H, t, J = 7.1 Hz), 3.95 (2H, s), 4.35 (2H, t, J = 7.6 Hz), 6.68 (1H, d, J = 9.5 Hz), 7.20 (1H, ddd, J = 8.0, 6.4 and 1.5 Hz), 7.31 (1H, ddd, J = 8.0, 5.8 and 2.0 Hz), 7.49 - 7.57 (5H, m), 7.65 (1H, d, J = 9.5 Hz), 7.92 (1H, dt, J = 8.0 and 1.0 Hz).
Mass spectrum FABMS, m/z 403 (M+1).

### Example 22 1-[3-[4-(2-Methoxybenzyl)-1-piperazinyl]-propyl]-2 (1H)-quinolinone

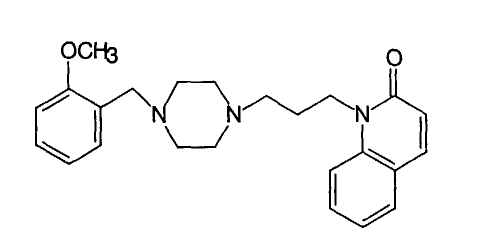

The title compound was synthesized in the same manner as in Example 1, except that 4-(2-methoxybenzyl)-1-piperidine was used instead of cyclohexanemethylpiperazine.
¹H NMR (CDCl₃) δ (ppm): 1.93 (2H, tt, J = 6.9 and 7.4 Hz), 2.43 - 2.65 (8H, m), 2.49 (2H, t, J = 6.9 Hz), 3.60 (2H, s), 3.82 (3H, s), 4.35 (2H, t, J = 7.4 Hz), 6.69 (1H, d, J = 9.5 Hz), 6.87 (1H, dd, J = 8.5 and 1.0 Hz), 6.93 (1H, dt, J = 1.0 and 7.5 Hz), 7.18 - 7.26 (2H, m), 7.35 (1H, dd, J = 7.5 and 1.8 Hz), 7.49 - 7.58 (3H, m), 7.66 (1H, d, J = 9.5 Hz).
Mass spectrum EIMS, m/z 391 (M⁺).

### Preparation Example 5 [3-[1-(Tert-butyloxy)carbonyl]-4-piperidinyl]-propyl]-2 (1H)-quinolinone

2-Hydroxyquinoline (145 mg) was dissolved in dimethylformamide (6 ml). 60% sodium hydride (40 mg) was added to the solution. The mixture was stirred at a room temperature for 30 min. 3-[1-(Tert-butyloxy)carbonyl]-4-piperidinyl]propyl iodide (350 mg), which may be produced from 4-pyridinepropanol according to a method described in a literature (J. Med. Chem., 37, 2537 (1994)), was added to the mixture, followed by heating with stirring at 60°C for 4 hr. The reaction solution was poured into an aqueous solution, and the mixture was extracted with dichloromethane. The extract was dried over anhydrous magnesium sulfate. The solvent was evaporated under the reduced pressure to give an oil. The oil was purified by column chromatography on silica gel to give 1-[3-(N-[(tert-butyloxy)carbonyl]-4-piperidinyl]-propyl]-2(1H)-quinolinone (252 mg).
¹H NMR (CDCL₃) δ (ppm): 1.06 - 1.15 (2H, m), 1.39 - 1.45 (3H, m), 1.45 (9H, s), 1.67 (2H, brd, J = 13.4 Hz), 1.73 - 1.82 (2H, m), 2.66 (2H, brt, J = 12.2 Hz), 4.07 (2H, brs), 4.27 (2H, t, J = 7.8 Hz), 6.69 (1H, d, J = 9.5 Hz), 7.22 (1H, t, J = 7.5 Hz), 7.33 (1H, d, J = 9.0 Hz), 7.53 - 7.58 (1H, m), 7.57 (1H, d, J = 7.5 Hz), 7.66 (1H, d, J = 9.5 Hz).
Mass spectrum EIMS, m/z 370 (M⁺).

### Preparation Example 6 1-[3-(4-Piperidinyl)-propyl]-2(1H)-quinolinone

Dichloromethane (2 ml) and trrluoroacetic acid (2 ml) were added to 1-[3-[N-[(tert-butyloxy)carbonyl]-4-piperidinyl]-propyl]-2(1H)-quinolinone (247 mg). The mixture was stirred at a room temperature for 4 hr. The solvent was evaporated under the reduced pressure. The residue was diluted with dichloromethane and washed with a saturated aqueous sodium hydrogencarbonate solution. The organic layer was dried over anhydrous magnesium sulfate. The solvent was evaporated under the reduced pressure to give 1-[3-(4-piperidinyl)-propyl]-2 (1H)-quinolinone (136 mg).
¹H NMR (CDCl₃) δ (ppm): 1.12 - 1.26 (2H,m), 1.38 - 1.50 (3H, m), 1.72 - 1.82 (4H, m), 2.61 (2H, dt, J = 2.5 and 12.2 Hz), 3.10 (2H, brd, J = 12.4 Hz), 4.27 (2H, t, J = 7.8 Hz), 6.69 (1H, d, J = 9.5 Hz), 7.22 (1H, t, J = 7.5 Hz), 7.34 (1H, d, J = 8.8 Hz), 7.54 - 7.59 (2H, m), 7.66 (1H, d, J = 9.5 Hz).
Mass spectrum EIMS, m/z 270 (M⁺).

### Example 23 1-[3-(1-Cyclohexanemethyl-4-piperidinyl)-propyl]-2(1H)-quinolinone

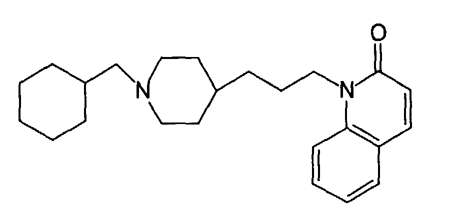

Cyclohexylmethyl bromide (47 mg), 1-[3-(4-piperidinyl)-propyl]-2(1H)-quinolinone (65 mg) obtained in Preparation Example 6, and potassium carbonate (50 mg) were suspended in dimethylformamide (1.5 ml). The suspension was stirred at a room temperature for 18 hr. Dichloromethane was added to the reaction solution. The mixture was washed with water and dried over anhydrous magnesium sulfate. The solvent was evaporated under the reduced pressure to give an oil. The oil was purified by column chromatography on silica gel to give the title compound (24 mg).
¹H NMR (CDCl₃) δ (ppm): 0.80 - 0.93 (3H, m), 1.05 - 1.55 (12H, m), 1.68 - 1.95 (7H, m), 2.10 (2H, brs), 2.86 (2H, brs), 4.26 (2H, t, J = 7.8 Hz), 6.69 (1H, d, J = 9.5 Hz), 7.22 (1H, t, J = 7.5 Hz), 7.33 (1H, d, J = 9.0 Hz), 7.56 - 7.58 (2H, m), 7.65 (1H, d, J = 9.5 Hz).

| High Mass (FAB, M+1): | |
|---|---|
| Calculated C₂₄H₃₅N₂O: | 367.2750. |
| Found: | 367.2744. |

### Example 24 1-[3-[1- (3,4-Difluorobenzyl)-4-piperidinyl]-propyl]-2 (1H)-quinolinone

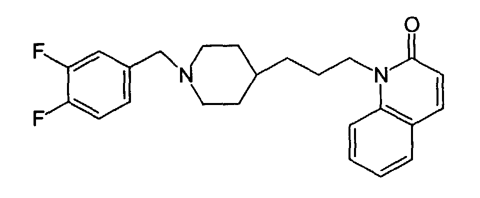

The title compound was synthesized in the same manner as in Example 23, except that 3,4-diiluorobenzyl bromide was used instead of cyclohexanemethyl bromide.
¹H NMR (CDCl₃) δ (ppm): 1.19 - 1.33 (3H, m), 1.37 - 1.45 (2H, m), 1.67 (2H, brd, J = 11.4 Hz), 1.71 - 1.80 (2H, m), 1.92 (2H, brt, J = 11.4 Hz), 2.81 (2H, brd, J = 11.4 Hz), 3.40 (2H, s), 4.26 (2H, t, J = 7.8 Hz), 6.74 (1H, d, J = 9.6 Hz), 6.98 - 7.02 (1H, m), 7.06 (1H, dt, J = 10.2 and 8.2 Hz), 7.15 (1H, ddd, J = 11.2, 8.0 and 2.0 Hz), 7.22 (1H, dt, J = 1.0 and 7.6 Hz) 7.33 (1H, d, J = 9.2 Hz), 7.55 (1H, dt, J = 1.6 and 7.2 Hz), 7.56 (1H, d, J = 7.6 Hz), 7.66 (1H, d, J = 9.6 Hz).

| High Mass (FAB, M+1): | |
|---|---|
| Calculated C₂₄H₂₇N₂OF₂: | 397.2091. |
| Found: | 397.2102. |

### Example 25 1-[3-[4-(4-Chlorophenoxy)-1-piperidinyl]-ethyl]-2 (1H)-quinolinone

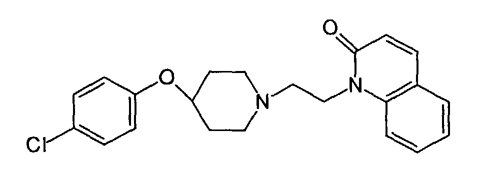

The title compound was synthesized in the same manner as in Example 8, except that 4-(4-chlorophenoxy)-1-piperidine was used instead of cyclohexanemethylpiperarine.
¹H NMR (CDCl₃) δ (ppm): 1.78 - 1.89 (2H, m), 1.96 - 2.05 (2H, m), 2.44 - 2.52 (2H, m), 2.70 (2H, brt, J = 7.7 Hz), 2.85 - 2.95 (2H, m), 4.29 (1H, tt, J = 7.7 and 3.7 Hz), 4.46 (2H, t, J = 7.7 Hz), 6.70 (1H, d, J = 9.5 Hz), 6.84 (2H, d, J = 9.0 Hz), 7.24 (2H, d, J = 9.0 Hz), 7.21 - 7.25 (1H, m), 7.44 (1H, d, J = 8.8 Hz), 7.54 - 7.60 (2H, m), 7.67 (1H, d, J = 9.5 Hz).;

| High Mass (FAB, M+1): | |
|---|---|
| Calculated C₂₂ H₂₄N₂ O₂Cl: | 383.1527. |
| Found: | 383.1532. |

### Preparation Example 7 Tert-butyl 4-[3- (methylsulfonyloxy)propyl] piperidinecarboxylate

3-[N-[(Tert-butyloxy)carbonyl]-4-piperidinyl]propanol (246 mg) synthesizd from 4-pyridinepropanol by a method described in a literature (J. Med. Chem., 37, 2537 (1994) was dissolved in anhydrous tetrahydrofuran (1.5 ml). Diisopropylethylamine (323 mg) and dimethylamino-pyridine (6.1 mg) were added to the solution. The mixture was cooled to 0°C. Methanesulfonyl chloride (137 mg) was added dropwise thereto, followed by stirring at 0°C for 45 min. The reaction solution was diluted with dictuoromethane, washed with water, and dried over anhydrous magnesium sulfate. The solvent was evaporated under the reduced pressure. The residue was purified by column chromatography on silica gel to give the title compound (387 mg).
¹H NMR (CDCl₃) δ (ppm): 1.04 - 1.16 (2H, m), 1.29 - 1.48 (3H, m), 1.45 (9H, s), 1.65 (2H, brd, J = 12.7 Hz), 1.78 (2H, tt, J = 6.8 and 6.6 Hz), 2.67 (2H, brt, J = 12.7 Hz), 3.00 (3H, s), 4.08 (2H, brs), 4.22 (2H, t, J = 6.6 Hz).

| High Mass (FAB, M+1): | |
|---|---|
| Calculated C₁₄H₂₈NO₅S: | 322.1688. |
| Found: | 322.1689. |

### Preparation Example 8 [3-[N-[(Tert-butyloxy)carboxyl]-4-piperidinyl]-propyl]-4H-benzo[1,4]oxazin-3-one

Benzo[1,4]oxazin-3-one (1.49 g) was dissolved in dimethylformamide (20 ml). 60% sodium hydride (600 mg) was added to the solution. The mixture was stirred at a room temperature for 2 hr. A solution of tert-butyl 4-[3-(methylsulfonyloxy)propyl]piperidinecarboxylate (3.21 g), obtained in Preparation Example 7, in dimethylformamide (11 ml) was added thereto. The mixture was stirred at 60°C for 2 hr and then at a room temperature for 12 hr. The reaction solution was poured into an aqueous solution. The mixture was extracted with dichloromethane. The extract was dried over anhydrous magnesium sulfate. The solvent was evaporated under the reduced pressure. The residue was purified by column chromatography on silica gel to give the title compound (2.94 g).
¹H NMR (CDCl₃) δ (ppm): 1.03 - 1.13 (2H, m), 1.24 - 1.48 (3H, m), 1.45 (9H, s), 1.62 - 1.73 (4H, m), 2.65 (2H, brt, J = 12.5 Hz), 3.93 (2H, t, J = 7.7 Hz), 4.05 (2H, brs), 4.59 (2H, s), 6.95 - 7.06 (4H, m).
Mass spectrum EIMS, m/z 374 (M⁺).

### Preparation Example 9 1-[3-(4-Piperidinyl)-propyl]-4H-benzo[1,4]oxazin-3-one

10% Hydrochloric acid/methanol solution (30 ml) was added to [3-[N-[(tert-butyloxy)carbonyl]-4-piperidinyl]-propyl]-4H-benzo[1,4]oxazin-3-one (2.85 g). The mixture was stirred at a room temperature for 2 hr. The solvent was evaporated under the reduced pressure. The residue was diluted with dichloromethane, washed with a saturated aqueous sodium hydrogencarbonate solution, and then dried over anhydrous magnesium sulfate. The solvent was evaporated under the reduced pressure to give 1-[3-(4-piperidinyl)-propyl]-4H-benzo[1,4]oxazin-3-one (2.06 g).
¹H NMR (CDCl₃) δ (ppm): 1.03 - 1.15 (2H, m), 1.27 - 1.43 (3H, m), 1.62 - 1.74 (4H, m), 2.56 (2H, dt, J = 2.2 and 12.2 Hz), 3.05 (2H, d, J = 12.2 Hz), 3.91 (2H, t, J = 7.7 Hz), 4.59 (2H, s), 6.96 - 7.06 (4H, m).
Mass spectrum EIMS, m/z 274 (M⁺).

### Example 26 1-[3-[1- (3, 4-Difluoroberzyl)-4-piperidinyl]-propyl]-4H-benzo[1, 4]oxazin-3-one

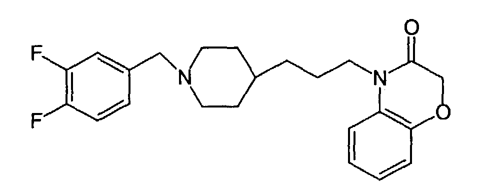

3,4-Difluorobenzaldehyde (35 mg) was dissolved in 1,2-dichloroethane (1.0 ml). 1-[3-(4-Piperidinyl)-propyl]-4H-benzo[1,4]oxazin-3-one (68 mg) obtained in Preparation Example 9, sodium triacetoxyboride (106 mg), and acetic acid (0.03 ml) were added in that order to the solution. The mixture was stirred at a room temperature for 15 hr. The reaction solution was evaporated under the reduced pressure. Dichloromethane was added to the residue. The mixture was washed with a saturated aqueous sodium hydrogencarbonate solution. The organic layer was dried over anhydrous magnesium sulfate. The solvent was evaporated under the reduced pressure. The residue was purified by column chromatography on silica gel to give the title compound (93 mg).
¹H NMR (CDCl₃) δ (ppm): 1.19 - 1.36 (5H, m), 1.60 - 1.72 (4H, m), 1.91 (2H, t, J = 11.2 Hz), 2.80 (2H, d, J = 11.7 Hz), 3.40 (2H, s), 3.90 (2H, t, J = 7.7 Hz), 4.58 (2H, s), 6.94 - 7.11 (6H, m), 7.12 - 7.19 (1H, m).

| High Mass (FAB, M+1): | |
|---|---|
| Calculated C₂₃ H₂₇N₂O₂F₂: | 401.2041. |
| Found: | 401.2018. |

### Example 27 1-[3-[1-(4-Fluorobenzyl)-4-piperidinyl]-propyl]-4H-benzo[1,4]oxazin-3-one

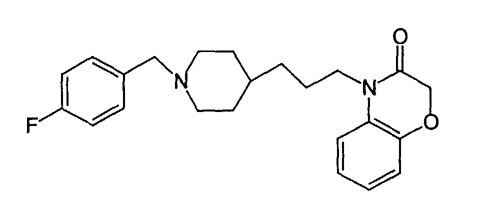

The title compound was synthesized in the same manner as in Example 26, except that 4-fluorobenzaldehyde was used instead of 3,4-difuorobenzaldehyde.
¹H NMR (CDCl₃) δ (ppm): 1.16 - 1.36 (5H, m), 1.59 - 1.71 (4H, m), 1.90 (2H, t, J = 11.0 Hz), 2.82 (2H, d, J = 11.5 Hz), 3.42 (2H, s), 3.90 (2H, t, J = 7.7 Hz), 4.58 (2H, s), 6.93 - 7.08 (6H, m), 7.22 - 7.28 (2H, m).

| High Mass (FAB, M+1): | |
|---|---|
| Calculated C₂₃ H₂₈N₂O₂F: | 383.2135. |
| Found: | 383.2133. |

### Example 28 1-[3-[1-(3-Fluorobenzyl)-4-piperidinyl]-propyl]-4H-benzo[1, 4]oxazin-3-one

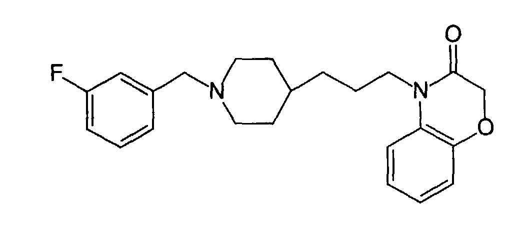

The title compound was synthesized in the same manner as in Example 26, except that 3-fluorobenzaldehyde was used instead of 3,4-difluorobenzaldehyde.
¹H NMR (CDCl₃) δ (ppm): 1.18 - 1.36 (5H, m), 1.58 - 1.72 (4H, m), 1.92 (2H, t, J = 11.0 Hz), 2.83 (2H, d, J = 11.2 Hz), 3.45 (2H, s), 3.90 (2H, t, J = 7.7 Hz), 4.58 (2H, s), 6.88 - 7.10 (7H, m), 7.21 - 7.28 (1H, m).

| High Mass (FAB, M+1): | |
|---|---|
| Calculated C₂₃H₂₈N₂O₂F: | 383.2134. |
| Found: | 383.2120. |

### Example 29 1-[3-[1-(4-Chlorobenzyl)-4-piperidinyl]-propyl]-4H-benzo[1,4]oxazin-3-one

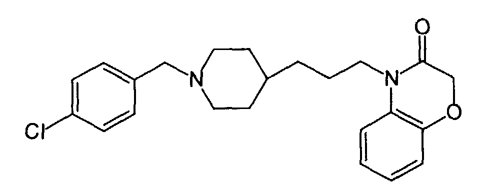

The title compound was synthesized in the same manner as in Example 26, except that 4-chlorobenzaldehyde was used instead of 3,4-difluorobenzaldehyde.
¹H NMR (CDCl₃) δ (ppm): 1.16 - 1.36 (5H, m), 1.58 - 1.72 (4H, m), 1.90 (2H, t, J = 11.0 Hz), 2.82 (2H, d, J = 11.5 Hz), 3.42 (2H, s), 3.90 (2H, t, J = 7.7 Hz), 4.58 (2H, s), 6.94 - 7.06 (4H, m), 7.21 - 7.30 (4H, m).;

| High Mass (FAB, M+1): | |
|---|---|
| Calculated C₂₃H₂₈N₂O₂Cl: | 399.1839. |
| Found: | 399.1857. |

### Preparation Example 10 [3-[N-[(Tert-butyloxy)carbonyl]-4-piperidinyl]-propyl]-3,4-dihydro-2 (1H)-quinolinone

The title compound was synthesized in the same manner as in Preparation Example 5, except that 3,4-dihydroquinolinone was used instead of 2-hydroxyquinoline.
¹H NMR (CDCl₃) δ (ppm): 1.01 - 1.13 (2H, m), 1.27 - 1.35 (2H, m), 1.35 - 1.50 (1H, m), 1.45 (9H, s), 1.57 - 1.70 (4H, m), 2.60 - 2.70 (4H, m), 2.85 - 2.92 (2H, m), 3.91 (2H, t, J = 7.7 Hz), 4.06 (2H, brs), 6.96 (1H, brd, J = 8.0 Hz), 6.99 (1H, dt, J = 1.0 and 7.5 Hz), 7.16 (1H, dd, J = 7.5 and 1.5 Hz), 7.24 (1H, ddd, J = 8.0, 7.5 and 1.5 Hz).

| High Mass (FAB, M+1): | |
|---|---|
| Calculated C₂₂H₃₃N₂O₃: | 373.2491. |
| Found: | 373.2512. |

### Preparation Example 11 1-[3-(4-Piperidinyl)-propyl]-3,4-dihydro-2(1H)-quinolinone

The title compound was synthesized in the same manner as in Preparation Example 6, except that [3-[N-[(tert-butyloxy)carbonyl]-4-piperidinyl]-propyl]-3,4-dihydro-2(1H)-quinolinone was used instead of 1-[3-[N-[(tert-butyloxy)carbonyl]-4-piperidinyl]-propyl]-2(1H)-quinolinone.
¹H NMR (CDCl₃) δ (ppm): 1.03 - 1.15 (2H, m), 1.24 - 1.44 (3H, m), 1.62 - 1.71 (4H, m), 2.55 (2H, dt, J = 2.4 and 12.2 Hz), 2.60 - 2.67 (2H, m), 2.88 (2H, dd, J = 8.1 and 6.8 Hz), 3.05 (2H, brd, J = 12.2 Hz), 3.90 (2H, t, J = 7.8 Hz), 6.99 (1H, d, J = 8.0 Hz), 7.00 (1H, dt, J = 1.0 and 7.5 Hz), 7.16 (1H, dd, J = 7.5 and 1.5 Hz), 7.24 (1H, ddd, J = 8.0, 7.5 and 1.5 Hz).

| High Mass (FAB, M+1): | |
|---|---|
| Calculated C₁₇ H₂₅N₂O: | 273.1967. |
| Found: | 273.1963. |

### Example 30 1-[3-[1- (3, 4-Difuorobenzyl)-4-piperidinyl]-propyl]-3,4-dihydro-2 (1H)-quinolinone

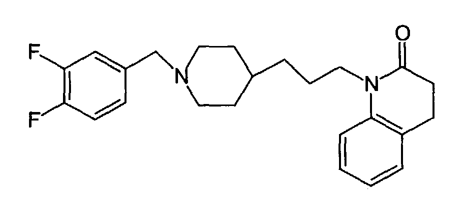

3,4-Difluorobenzaldehyde (30 mg) was dissolved in 1,2-dichloroethane (1.5 ml). 1-[3-(4-Piperidinyl)-propyl]-3,4-dihydro-2(1H)-quinolinone (57 mg) obtained in Preparation Example 11, sodium borohydrade (130 mg), and acetic acid (35 µl) were added in that order to the solution. The mixture was stirred at a room temperature for 15 hr. Dichloromethane was added to the reaction mixture. The mixture was washed with a saturated aqueous sodium hydrogencarbonate solution. The organic layer was dried over anhydrous magnesium sulfate. The solvent was evaporated under the reduced pressure to give an oil. The oil was purified by column chromatography on silica gel to give the title compound (44 mg).
¹H NMR (CDCl₃) δ (ppm): 1.18 - 1.36 (5H, m), 1.58 - 1.72 (4H, m), 1.97 (2H, brt, J = 11.0 Hz), 2.59 - 2.68 (2H, m), 2.81 - 2.92 (4H, m), 3.46 (2H, s), 3.90 (2H, t, J = 7.8 Hz), 6.96 (1H, brd, J = 8.2 Hz), 6.99 (1H, dt, J = 1.0 and 7.4 Hz), 7.01 - 7.13 (2H, m), 7.14 - 7.28 (3H, m).

| High Mass (FAB, M+1): | |
|---|---|
| Calculated C₂₄H₂₉N₂OF₂: | 399.2248. |
| Found: | 399.2241. |

### Preparation Example 12 [3-[N-[(Tert-butyloxy)carbonyl]-4-piperidinyl]-propyl]-4H-benzo[1, 4]thiazin-3-one

The title compound was synthesized in the same manner as in Preparation Example 5, except that benzo[1,4]thiazin-3-one was used instead of 2-hydroxyquinoline.
¹H NMR (CDCl₃) δ (ppm): 0.99 - 1.11 (2H, m), 1.24 - 1.31 (2H, m), 1.31 - 1.48 (1H, m), 1.44 (9H, s), 1.60 - 1.70 (4H, m), 2.62 (2H, brt, J = 12.2 Hz), 3.37 (2H, s), 4.00 (2H, t, J = 7.5 Hz), 4.10 (2H, brs), 7.02 (1H, dt, J = 1.2 and 7.6 Hz), 7.09 (1H, d, J = 7.6 Hz), 7.21 - 7.28 (1H, m), 7.37 (1H, dd, J = 7.6 and 1.4 Hz).
Mass spectrum EIMS, m/z 390 (M⁺).

### Preparation Example 13 1-[3-(4-Piperidinyl)-propyl]-4H-benzo[1,4] thiazin-3-one

The title compound was synthesized in the same manner as in Preparation Example 6, except that [3-[N-[(tert-butyloxy)carbonyl]-4-piperidinyl]-propyl]-4H-benzo[1,4]thiazin-3-one was used instead of 1-[3-[N-[(tert-butyloxy)carbonyl]-4-piperidinyl]-propyl]-2(1H)-quinolinone.
¹H NMR (CDCl₃) δ (ppm): 1.28 - 1.37 (2H, m), 1.41 - 1.52 (3H, m), 1.59 - 1.69 (2H, m), 1.79 (2H, brd, J = 9.7 Hz), 2.74 (2H, t, J = 11.5 Hz), 3.33 (2H, d, J = 11.7 Hz), 3.37 (2H, s), 3.99 (2H, t, J = 7.6 Hz), 7.02 (1H, ddd, J = 7.8, 7.3 and 1.0 Hz), 7.08 (1H, dd, J = 8.3 and 1.0 Hz), 7.24 (1H, ddd, J = 8.3, 7.3 and 1.4 Hz), 7.37 (1H, dd, J = 7.8 and 1.4 Hz).
Mass spectrum EIMS, m/z 290 (M⁺).

### Example 31 1-[3-[1-(3,4-difluorobenzyl)-4-piperidinyl]-propyl]-4H-benzo[1,4]thiazin-3-one

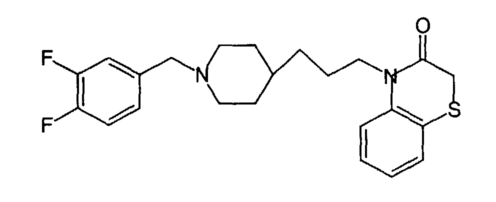

3,4-Difluorobenzaldehyde (75 mg) was dissolved in 1,2-dichloroethane (2 ml). 1-[3-(4-Piperidinyl)-propyl]-4H-benzo[1,4]thiaizin-3-one (149 mg) obtained in Preparation Example 13, sodium borohydrade (212 mg), and acetic acid (0.09 ml) were added in that order to the solution. The mixture was stirred at a room temperature for 15 hr. The reaction mixture was evaporated under reduced pressure. Dichloromethane was added to the residue. The mixture was washed with a saturated aqueous sodium hydrogencarbonate solution. The organic layer was dried over anhydrous magnesium sulfate. The solvent was evaporated under the reduced pressure to give an oil. The oil was purified by column chromatography on silica gel to give the tile compound (174 mg).
¹H MMR (CDCl₃) δ (ppm): 1.15 - 1.31 (5H, m), 1.59 - 1.68 (4H, m), 1.92 (2H, t, J = 11.0 Hz), 2.81 (2H, d, J = 11.5 Hz), 3.37 (2H, s), 3.41 (2H, s), 3.97 (2H, t, J = 7.5Hz), 6.97 - 7.08 (2H, m), 7.01 (1H, ddd, J = 7.5, 7.3 and 1.2 Hz), 7.09 (1H, dd, J = 8.3 and 1.2 Hz), 7.15 (1H, ddd, J = 11.3, 7.8 and 2.0 Hz), 7.23 (1H, ddd, J = 8.3, 7.3 and 1.5 Hz), 7.36 (1H, dd, J = 7.5 and 1.5 Hz).;

| High Mass (FAB, M+1): | |
|---|---|
| Calculated C₂₃H₂₇N₂OF₂S: | 417.1812. |
| Found: | 417.1809. |

### Example 32 1-[3-[1-(4-Fluorobenzyl)-4-piperidinyl]-propyl]-4H-benzo[1,4]thiazin-3-one

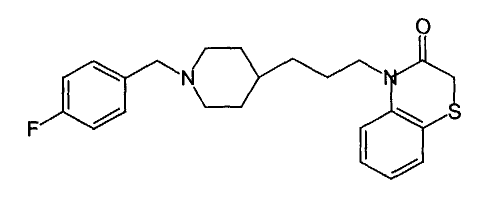

The title compound was synthesized in the same manner as in Example 31, except that 4-fluorobenzaldehyde was used instead of 3,4-difluorobenzaldehyde.
¹H NMR (CDCl₃) δ (ppm): 1.23 - 1.40 (5H, m), 1.58 - 1.69 (4H, m), 2.10 (2H, t, J = 11.0 Hz), 3.04 (2H, brd, J = 11.9 Hz), 3.36 (2H, s), 3.67 (2H, s), 3.97 (2H, t, J = 7.5 Hz), 6.80 - 7.04 (3H, m), 7.08 (1H, dd, J = 8.3 and 1.0 Hz), 7.23 (1H, ddd, J = 8.3, 7.6 and 1.5 Hz), 7.27 - 7.33 (2H, m), 7.36 (1H, dd, J = 7.8 and 1.5 Hz).

| High Mass (FAB, M+1) | |
|---|---|
| Calculated C₂₃H₂₈N₂OFS: | 399.1906. |
| Found: | 399.1920. |

### Example 33 1-[3-[1-(4-Chlorobenzyl)-4-piperidinyl]-propyl]-4H-benzo[1,4]thiazin-3-one

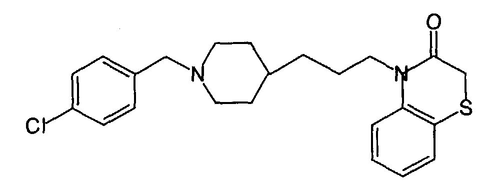

The title compound was synthesized in the same manner as in Example 31, except that 4-chlorobenzaldehyde was used instead of 3,4-difluorobenzaldehyde.
¹H NMR (CDCl₃) δ (ppm): 1.23 - 1.40 (5H, m), 1.58 - 1.70 (4H, m), 2.11 (2H, t, J = 11.5 Hz), 3.04 (2H, d, J = 11.7 Hz), 3.36 (2H, s), 3.67 (2H, s), 3.97 (2H, t, J = 7.5 Hz), 7.01 (1H, ddd, J = 7.6, 7.3 and 1.0 Hz), 7.08 (1H, dd, J = 8.3 and 1.0 Hz), 7.23 (1H, ddd, J = 8.3, 7.3 and 1.4 Hz), 7.24 - 7.32 (4H, m), 7.36 (2H, dd, J = 7.6 and 1.4 Hz).

| High Mass (FAB, M+1): | |
|---|---|
| Calculated C₂₃H₂₈N₂OClS: | 415.1611. |
| Found: | 415.1644. |

### Example 34 1-[3-[1-(4-Trifluoromethylbenzyl)-4-piperidinyl]-propyl]-4H-benzo[1,4]thiazin-3-one

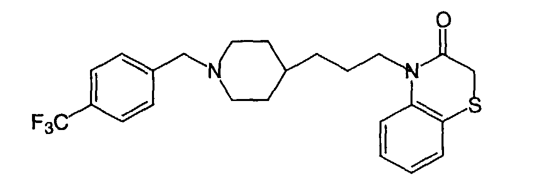

The title compound was synthesized in the same manner as in Example 31, except that 4-trifluoromethylbenzaldehyde was used instead of 3,4-difluorobenzaldehyde.
¹H NMR (CDCl₃) δ (ppm): 1.22 - 1.43 (5H, m), 1.57 - 1.72 (4H, m), 2.15 (2H, t, J = 11.0 Hz), 3.09 (2H, d, J = 11.2 Hz), 3.36 (2H, s), 3.79 (2H, s), 3.97 (2H, t, J = 7.6 Hz), 7.01 (1H, ddd, J = 7.8, 7.6 and 1.0 Hz), 7.08 (1H, dd, J = 8.3 and 1.0 Hz), 7.23 (1H, ddd, J = 8.3, 7.6 and 1.5 Hz), 7.36 (1H, dd, J = 7.8 and 1.5 Hz), 7.47 (2H, d, J = 8.1 Hz), 7.59 (2H, d, J = 8.1 Hz).

| High Mass (FAB, M+1): | |
|---|---|
| Calculated C₂₄H₂₈N₂OF₃S: | 449.1874. |
| Found: | 449.1856. |

### Example 35 1-[3-[1-(4-Methoxybenzyl)-4-piperidinyl]-propyl]-4H-benzo[1,4]thiazin-3-one

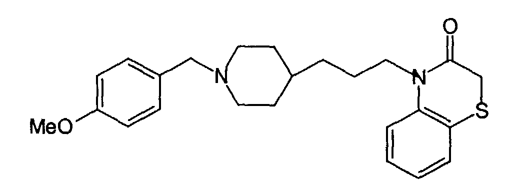

The title compound was synthesized in the same manner as in Example 31, except that 4-methoxybenzaldehyde was used instead of 3,4-difluorobenzaldehyde.
¹H NMR (CDCl₃) δ (ppm): 1.20 - 1.39 (5H, m), 1.58 - 1.67 (4H, m), 2.01 (2H, t, J = 11.0 Hz), 2.96 (2H, d, J = 11.7 Hz), 3.36 (2H, s), 3.56 (2H, s), 3.80 (3H, s), 3.96 (2H, t, J = 7.5 Hz), 6.85 (2H, d, J = 8.8 Hz), 7.01 (1H, ddd, J = 7.8, 7.6 and 1.0 Hz), 7.08 (1H, dd, J = 8.3 and 1.0 Hz), 7.23 (1H, ddd, J = 8.3, 7.6 and 1.5 Hz) 7.23 (2H, d, J = 8.8 Hz), 7.36 (1H, dd, J = 7.8 and 1.5 Hz).

| High Mass (FAB, M + 1): | |
|---|---|
| Calculated C₂₄H₃₁N₂O₂S: | 411.2106. |
| Found: | 411.2131. |

### Example 36 1-[3-[1-(4-Methylbenzyl)-4-piperidinyl]-propyl]-4H-benzo[1,4]thiazin-3-one

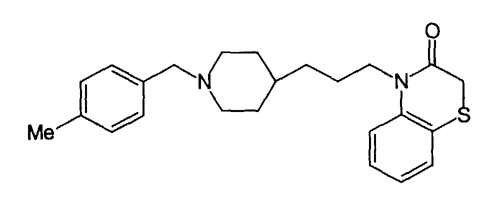

The title compound was synthesized in the same manner as in Example 31, except that 4-methylbenzaldehyde was used instead of 3,4-difluorobenzaldehyde.
¹H NMR (CDCl₃) δ (ppm): 1.17 - 1.30 (5H, m), 1.58 - 1.67 (4H, m), 1.90 (2H, m), 2.32 (3H, s), 2.85 (2H, d, J = 11.0 Hz), 3.36 (2H, s), 3.45 (2H, s), 3.96 (2H, t, J = 7.5 Hz), 7.00 (1H, ddd, J = 7.8, 7.3 and 1.2 Hz), 7.08 (1H, dd, J = 8.3 and 1.2 Hz), 7.11 (2H, d, J = 7.8 Hz), 7.18 (2H, d, J = 7.8 Hz), 7.23 (1H, ddd, J = 8.3, 7.3 and 1.4 Hz), 7.36 (1H, dd, J = 7.8 and 1.4 Hz).

| High Mass (FAB, M+1): | |
|---|---|
| Calculated C₂₄N₃₁N₂OS: | 395.2172. |
| Found: | 395.2147. |

### Example 37 1-[3-[1-(2-Methylbenzyl)-4-piperidinyl]-propyl]-4H-benzo[1,4]oxazin-3-one

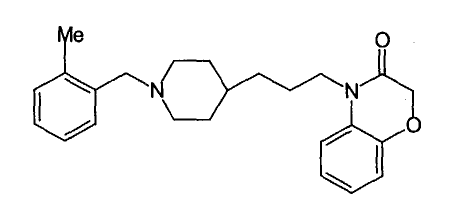

The title compound was synthesized in the same manner as in Example 26, except that 2-methylbenzaldehyde was used instead of 3,4-difluorobenzaldehyde.
¹H NMR (CDCl₃) δ (ppm): 1.14 - 1.35 (5H, m), 1.55 - 1.71 (4H, m), 1.93 (2H, t, J = 11.2 Hz), 2.34 (3H, s), 2.83 (2H, d, J = 11.7 Hz), 3.40 (2H, s), 3.90 (2H, t, J = 7.8 Hz), 4.58 (2H, s), 6.94 - 7.06 (4H, m), 7.10 - 7.15 (3H, m), 7.23 - 7.25 (1H, m).

| High Mass (FAB, M+1): | |
|---|---|
| Calculated C₂₄N₃₁N₂O₂: | 379.2385. |
| Found: | 379.2357. |

### Example 38 1-[3-[1-Benzyl-4-piperidinyl]-propyl]-3,4-dihydro-2(1H)-quinolinone

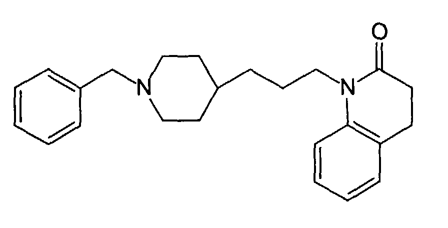

The title compound was synthesized in the same manner as in Example 30, except that benzaldehyde was used instead of 3,4-difluorobenzaldehyde.
¹H NMR (CDCl₃) δ (ppm): 1.18 - 1.33 (5H, m), 1.59 - 1.69 (4H, m), 1.91 (2H, t, J = 11.0 Hz), 2.60 - 2.65 (2H, m), 2.82 - 2.90 (4H, m), 3.47 (2H, s), 3.99 (2H, t, J = 7.8 Hz), 6.96 (1H, brd, J = 7.8 Hz), 6.99 (1H, ddd, J = 7.6, 7.3 and 1.0 Hz), 7.15 (1H, dd, J = 7.6 and 1.5 Hz), 7.20 - 7.33 (6H, m).

| High Mass (FAB, M+1): | |
|---|---|
| Calculated C₂₄H₃₁N₂O: | 363.2436. |
| Found: | 363.2447. |

### Example 39 1-[3-[1-(2,4-Difluorobenzyl)-4-piperidinyl]propyl]-3,4-dihydro-2(1H)-quinolinone

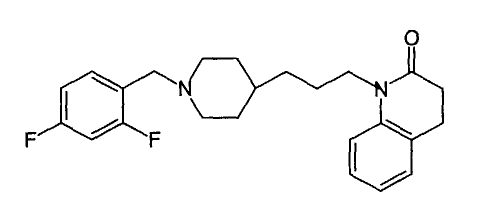

The title compound was synthesized in the same manner as in Example 30, except that 2,4-difluorobenzaldehyde was used instead of 3,4-difluorobenzaldehyde.
¹H NMR (CDCl₃) δ (ppm): 1.16 - 1.33 (5H, m), 1.59 - 1.68 (4H, m), 1.96 (2H, t, J = 11.0 Hz), 2.60 - 2.65 (2H, m), 2.81 - 2.91 (4H, m), 3.50 (2H, s), 3.89 (2H, t, J = 7.8 Hz), 6.74 - 6.92 (2H, m), 6.96 (1H, brd, J = 8.3 Hz), 6.99 (1H, ddd, J = 7.5, 7.3 and 1.0 Hz), 7.15 (1H, dd, J = 7.5 and 1.7 Hz), 7.23 (1H, ddd, J = 8.3, 7.3 and 1.7 Hz), 7.29 - 7.44 (1H, m).

| High Mass (FAB, M+1): | |
|---|---|
| Calculated C₂₄H₂₉N₂OF₂: | 399.2248. |
| Found: | 399.2240. |

### Example 40 1-[3-[1-(4-Fluorobenzyl)-4-piperidinyl]-propyl]-3,4-dihydro-2 (1H)-quinolinone

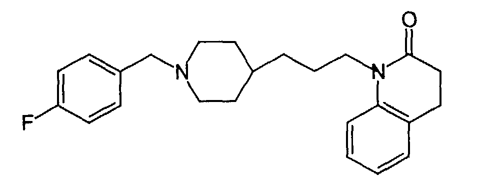

The title compound was synthesized in the same manner as in Example 30, except that 4-fluorobenzaldehyde was used instead of 3,4-difluorobenzaldehyde.
¹H NMR (CDCl₃) δ (ppm): 1.16 - 1.33 (5H, m), 1.60 - 1.68 (4H, m), 1.89 (2H, t, J = 11.2 Hz), 2.60 - 2.66 (2H, m), 2.79 - 2.90 (4H, m), 3.42 (2H, s), 3.89 (2H, t, J = 7.8 Hz), 6.94 - 7.07 (4H, m), 7.15 (1H, dd, J = 7.5 and 1.5 Hz), 7.20 - 7.28 (3H, m).

| High Mass (FAB, M+1): | |
|---|---|
| Calculated C₂₄H₃₀N₂OF: | 381.2342. |
| Found: | 381.2350. |

### Example 41 1-[3-[1-(4-Chlorobenzyl)-4-piperidinyl]-propyl]-3, 4-dihydro-2 (1H)-quinolinone

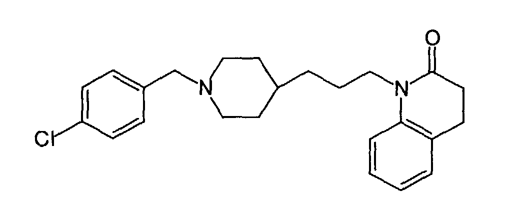

The title compound was synthesized in the same manner as in Example 30, except that 4-chlorobenaaldehyde was used instead of 3,4-difluorobenzaldehyde.
¹H NMR (CDCl₃) δ (ppm): 1.15 - 1.33 (5H, m), 1.59 - 1.68 (4H, m), 1.90 (2H, t, J = 11.0 Hz), 2.59 - 2.65 (2H, m), 2.77 - 2.84 (2H, brd, J = 11.5 Hz), 2.84 - 2.90 (2H, m), 3.42 (2H, s), 3.89 (2H, t, J = 7.8 Hz), 6.96 (1H, brd, J = 8.3 Hz), 6.99 (1H, ddd, J = 7.5, 7.3 and 1.0 Hz), 7.15 (1H, dd, J = 7.5 and 1.5 Hz), 7.21 - 7.34 (5H, m).

| High Mass (FAB, M+1): | |
|---|---|
| Calculated C₂₄H₃₀N₂OCl: | 397.2047. |
| Found: | 397.2040. |

### Example 42 1-[3-[1-(4-Trifluoromethylbenzyl)-4-piperidinyl]-propyl]-3,4-dihydro-2(1H)-quinolinone

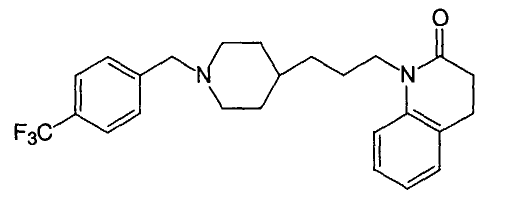

The title compound was synthesized in the same manner as in Example 30, except that 4-trifluoromethylbenzaldehyde was used instead of 3,4-difluorobenzaldehyde.
¹H NMR (CDCl₃) δ (ppm): 1.17 - 1.34 (5H, m), 1.60 - 1.69 (4H, m), 1.93 (2H, t, J = 11.2 Hz), 2.60 - 2.66 (2H, m), 2.82 (2H, brd, J = 11.4 Hz), 2.85 - 2.90 (2H, m), 3.50 (2H, s), 3.90 (2H, t, J = 7.8 Hz), 6.96 (1H, brd, J = 8.3 Hz), 6.99 (1H, brt, J = 7.3 Hz), 7.16 (1H, brd, J = 7.3 Hz), 7.23 (1H, ddd, J = 8.3, 7.3 and 1.5 Hz), 7.42 (2H, d, J = 8.3 Hz), 7.55 (2H, d, J = 8.3 Hz).

| High Mass (FAB, M+1): | |
|---|---|
| Calculated C₂₅H₃₀N₂OF₃: | 431.2310. |
| Found: | 431.2317. |

### Example 43 1-[3-[1-(4-Methoxybenzyl)-4-piperidinyl]propyl]-3,4-dihydro-2(1H)-quinolinone

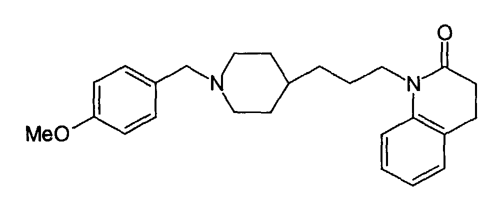

The title compound was synthesized in the same manner as in Example 30, except that 4-methoxybenzaldehyde was used instead of 3,4-difluorobenzaldehyde.
¹H NMR (CDCl₃) δ (ppm): 1.15 - 1.34 (5H, m), 1.59 - 1.68 (4H, m), 1.88 (2H, t, J = 11.0 Hz), 2.60 - 2.65 (2H, m), 2.81 - 2.90 (4H, m), 3.41 (2H, s), 3.79 (3H, s), 3.89 (2H, t, J = 7.6 Hz), 6.84 (2H, d, J = 8.5 Hz), 6.96 (1H, brd, J = 8.3 Hz), 6.99 (1H, dt, J = 1.0 and 7.5 Hz), 7.15 (1H, brd, J = 7.5 Hz), 7.21 (2H, d, J = 8.5 Hz), 7.23 (1H, ddd, J = 8.3, 7.5 and 1.5 Hz);

| High Mass (FAB, M+1): | |
|---|---|
| Calculated C₂₅H₃₃N₂O₂: | 393.2542 |
| Found: | 393.2548. |

### Example 44 1-[3-[1-(2-Methylbenzyl)-4-piperidinyl]-propyl]-3,4-dihydro-2(1H)-quinolinone

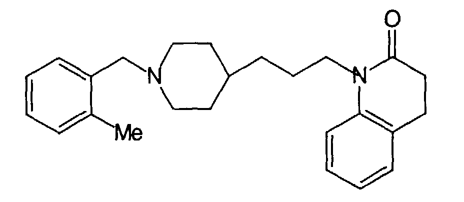

The title compound was synthesized in the same manner as in Example 30, except that 2-methylbenzaldehyde was used instead of 3,4-difluorobenzaldehyde.
¹H NMR (CDCl₃) δ (ppm): 1.14 - 1.33 (5H, m), 1.59 - 1.69 (4H, m), 1.93 (2H, t, J = 11.0 Hz), 2.36 (3H, s), 2.60 - 2.66 (2H, m), 2.80 - 2.90 (4H, m), 3.40 (2H, s), 3.90 (2H, t, J = 7.7 Hz), 6.96 (1H, d, J = 8.0 Hz), 6.99 (1H, ddd, J = 7.5, 7.3 and 1.0 Hz), 7.10 - 7.24 (6H,m).

| High Mass (FAB, M+1): | |
|---|---|
| Calculated C₂₅H₃₃N₂O: | 377.2593. |
| Found: | 377.2598. |

### Example 45 1-[3-[1-(4-Nitrilebenzyl)-4-piperidinyl]-propyl]-4H-benzo [1, 4]oxazin-3-one

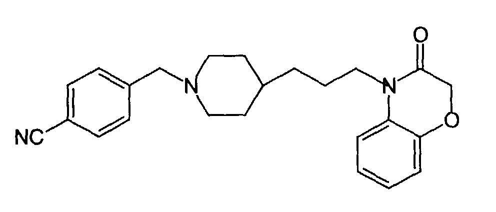

The title compound was synthesized in the same manner as in Example 26, except that 4-nitrilebenzaldehyde was used instead of 3,4-difluorobenzaldehyde.
¹H NMR (CDCl₃) δ (ppm): 1.18 - 1.36 (5H, m), 1.61 - 1.72 (4H, m), 1.95 (2H, t, J = 11.2 Hz), 2.79 (2H, d, J = 11.2 Hz), 3.50 (2H, s), 3.90 (2H, t, J = 7.8 Hz), 4.58 (2H, s), 6.94 - 7.05 (4H, m), 7.43 (2H, d, J = 8.0 Hz), 7.59 (2H, d, J = 8.0 Hz).

| High Mass (FAB, M+1): | |
|---|---|
| Calculated C₂₄ H₂₈N₃O₂: | 390.2182. |
| Found: | 390.2178. |

### Example 46 1-[3-[1-(4-Methoxybenzyl)-4-piperidinyl]-propyl]-4H-benzo[1,4]oxazin-3-one

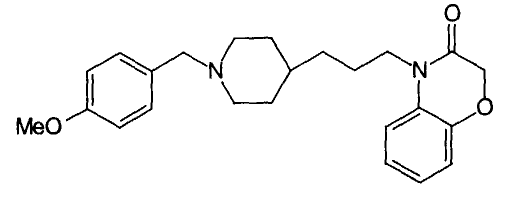

The title compound was synthesized in the same manner as in Example 26, except that 4-methoxybenzaldehyde was used instead of 3,4-difluorobenzaldehyde.
¹H NMR (CDCl₃) δ (ppm): 1.19 - 1.34 (5H, m), 1.58 - 1.71 (4H, m), 1.88 (2H, brt, J = 11.2 Hz), 2.84 (2H, d, J = 11.2 Hz), 3.41 (2H, s), 3.79 (3H, s), 3.89 (2H, t, J = 7.8 Hz), 4.58 (2H, s), 6.84 (2H, d, J = 8.5 Hz), 6.93 - 7.05 (4H, m), 7.21 (2H, d, J = 8.5 Hz).

| High Mass (FAB, M+1): | |
|---|---|
| Calculated C₂₄H₃₁N₂O₃: | 395.2334. |
| Found: | 395.2344. |

### Example 47 1-[3-[1-(4-Methylbenzyl)-4-piperidinyl]-propyl]-4H-benzo [1,4]oxazin-3-one

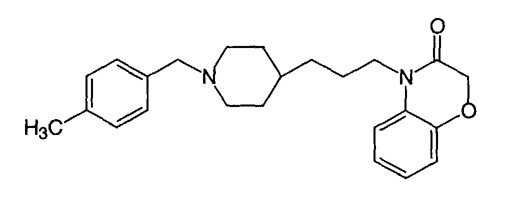

The title compound was synthesized in the same manner as in Example 26, except that 4-methylbenzaldehyde was used instead of 3,4-difluorobenzaldehyde.
¹H NMR (CDCl₃) δ (ppm): 1.18 - 1.34 (5H, m), 1.58 - 1.70 (4H, m), 1.89 (2H, brt, J = 11.0 Hz), 2.33 (3H, s), 2.84 (2H, d, J = 11.2 Hz), 3.43 (2H, s), 3.89 (2H, t, J = 7.8 Hz), 4.58 (2H, s), 6.94 - 7.04 (4H, m), 7.11 (2H, d, J = 7.8 Hz), 7.18 (2H, d, J = 7.8 Hz).

| High Mass (FAB, M+1): | |
|---|---|
| Calculated C₂₄N₃₁N₂O₂: | 379.2385. |
| Found: | 379.2403. |

### Example 48 1-[3-[1-(4-Trifluoromethylbenzyl)-4-piperidinyl]-propyl]-4H-benzo[1, 4]oxazin-3-one

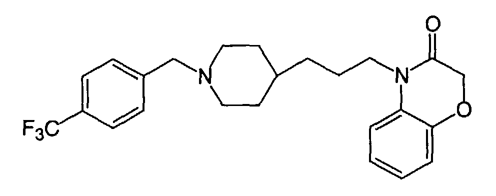

The title compound was synthesized in the same manner as in Example 26, except that 4-trifluoromethylbenzaldehyde was used instead of 3,4-difluorobenzaldehyde.
¹H NMR (CDCl₃) δ (ppm): 1.15 - 1.36 (5H, m), 1.59 - 1.72 (4H, m), 1.94 (2H, t, J = 11.0 Hz), 2.82 (2H, d, J = 11.2 Hz), 3.51 (2H, s), 3.90 (2H, t, J = 8.0 Hz), 4.58 (2H, s), 6.94 - 7.05 (4H, m), 7.42 (2H, d, J = 8.0 Hz), 7.55 (2H, d, J = 8.0 Hz).

| High Mass (FAB, M+1): | |
|---|---|
| Calculated C₂₄H₂₈N₂O₂F₃: | 433.2103. |
| Found: | 433.2105. |

### Example 49 1-[3-[1-(4-Isopropylbenzyl)-4-piperidinyl]-propyl]-4H-benzo[1,4]oxazin-3-one

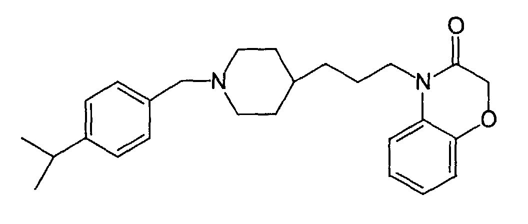

The title compound was synthesized in the same manner as in Example 26, except that 4-isopropylbenzaldehyde was used instead of 3,4-difluorobenzaldehyde.
¹H NMR (CDCl₃) δ (ppm): 1.19 - 1.34 (5H, m), 1.24 (6H, d, J = 6.9 Hz), 1.62 - 1.71 (4H, m), 1.90 (2H, brt, J = 11.0 Hz), 2.83 - 2.93 (3H, m), 3.44 (2H, s), 3.89 (2H, t, J = 7.8 Hz), 4.58 (2H, s), 6.96 - 7.05 (4H, m), 7.15 (2H, d, J = 8.0 Hz), 7.21 (2H, d, J = 8.0 Hz).

| High Mass (FAB, M+1): | |
|---|---|
| Calculated C₂₆H₃₅N₂O₂: | 407.2698. |
| Found: | 407.2687. |

### Example 50 1-[3-[1-(4-Tert-butylbenzyl)-4-piperidinyl]-propyl]-4H-benzo[1,4]oxazin-3-one

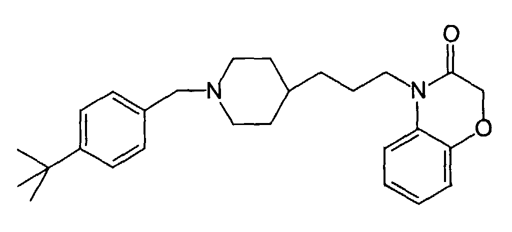

The title compound was synthesized in the same manner as in Example 26, except that 4-tert-butylbenzaldehyde was used instead of 3,4-difluorobenzaldehyde.
¹H NMR (CDCl₃) δ (ppm): 1.17 - 1.34 (5H, m), 1.31 (9H, s), 1.58 - 1.71 (4H, m), 1.90 (2H, brt, J = 11.0 Hz), 2.86 (2H, d, J = 11.0 Hz), 3.44 (2H, s), 3.89 (2H, t, J = 7.6 Hz), 4.58 (2H, s), 6.92 - 7.05 (4H, m), 7.22 (1H, d, J = 8.0 Hz), 7.32 (2H, d, J = 8.0 Hz).

| High Mass (FAB, M+1): | |
|---|---|
| Calculated C₂₇H₃₇N₂O₂: | 421.2899. |
| Found: | 421.2877. |

### Example 51 1-[3-[1-(2-Fluorobenzyl)-4-piperidinyl]-propyl]-4H-benzo [1,4]oxazin-3-one

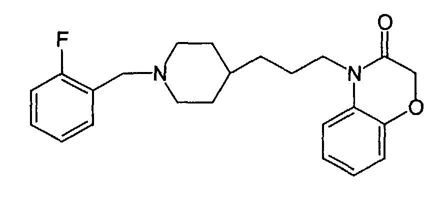

The title compound was synthesized in the same manner as in Example 26, except that 2-fluorobenzaldehyde was used instead of 3,4-difluorobenzaldehyde.
¹H NMR (CDCl₃) δ (ppm): 1.20 - 1.37 (5H, m), 1.62 - 1.70 (4H, m), 1.98 (2H, brt, J = 11.0 Hz), 2.87 (2H, d, J = 10.7 Hz), 3.56 (2H, s), 3.89 (2H, t, J = 7.7 Hz), 4.58 (2H, s), 6.94 - 7.03 (5H, m), 7.09 (1H, t, J = 7.4 Hz), 7.19 - 7.25 (1H, m), 7.34 - 7.38 (1H, m).

| High Mass (FAB, M+1): | |
|---|---|
| Calculated C₂₃ N₂₈N₂O₂F: | 383.2135. |
| Found: | 383.2138. |

### Example 52 1-[3-[1-(2-Chlorobenzyl)-4-piperidinyl]-propyl]-4H-benzo [1,4]oxazin-3-one

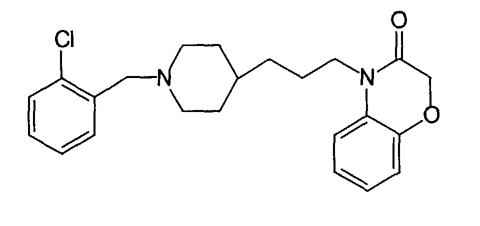

The title compound was synthesized in the same manner as in Example 26, except that 2-chlorobenzaldehyde was used instead of 3,4-difluorobenzaldehyde.
¹H NMR (CDCl₃) δ (ppm): 1.20 - 1.36 (5H, m), 1.62 - 1.73 (4H, m), 2.04 (2H, t, J = 11.0 Hz), 2.88 (2H, d, J = 11.7 Hz), 3.58 (2H, s), 3.90 (2H, t, J = 7.7 Hz), 4.58 (2H, s), 6.94 - 7.06 (4H, m), 7.16 (1H, ddd, J = 7.8, 7.6 and 2.0 Hz), 7.22 (1H, ddd, J = 7.6, 7.3 and 1.4 Hz), 7.32 (1H, dd, J = 7.8 and 1.4 Hz), 7.47 (1H, dd, J = 7.3 and 2.0 Hz).

| High Mass (FAB, M+1): | |
|---|---|
| Calculated C₂₃H₂₈N₂O₂Cl: | 399.1839. |
| Found: | 399.1834. |

### Example 53 1-[3-[1-(2-Methoxybenzyl)-4-piperidinyl]-propyl]-4H-benzo[1,4]oxazin-3-one

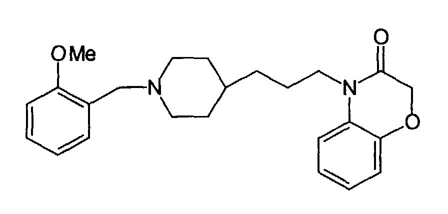

The title compound was synthesized in the same manner as in Example 26, except that 2-methoxybenzaldehyde was used instead of 3,4-difluorobenzaldehyde.
¹H NMR (CDCl₃) δ (ppm): 1.20 - 1.35 (5H, m), 1.64 - 1.74 (4H, m), 1.99 (2H, brt, J = 11.0 Hz), 2.90 (2H, d, J = 11.4 Hz), 3.53 (2H, s), 3.81 (3H, s), 3.89 (2H, t, J = 7.8 Hz), 4.58 (2H, s), 6.85 (1H, dd, J = 7.5 and 0.7 Hz), 6.92 (1H, dt, J = 0.7 and 7.5 Hz), 6.94 - 7.05 (4H, m), 7.21 (1H, dt, J = 1.7 and 7.5 Hz), 7.34 (1H, dd, J = 7.5 and 1.7 Hz).

| High Mass (FAB, M+1): | |
|---|---|
| Calculated C₂₄H₃₁N₂O₃: | 395.2334. |
| Found: | 395.2344. |

### Example 54 1-[3-[1-(3-Methylbenzyl)-4-piperidinyl]-propyl]-3,4-dihydro-2(1H)-quinolinone

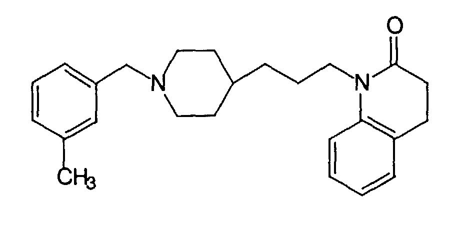

The title compound was synthesized in the same manner as in Example 30, except that 3-methylbenzaldehyde was used instead of 3,4-difluorobenzaldehyde.
¹H NMR (CDCl₃) δ (ppm): 1.18 - 1.33 (5H, m), 1.59 - 1.68 (4H, m), 1.90 (2H, brt, J = 11.0 Hz), 2.32 (3H, s), 2.60 - 2.65 (2H, m), 2.81 - 2.90 (4H, m), 3.44 (2H, s), 3.89 (2H, t, J = 7.8 Hz), 6.96 (1H, brd, J = 8.3 Hz), 6.99 (1H, dt, J = 7.3 and 1.0 Hz), 7.11 (2H, d, J = 7.8 Hz), 7. 15 (1H, brd, J = 7.3 Hz), 7.18 (2H, d, J = 7.8 Hz), 7.23 (1H, ddd, J = 8.3, 7.3 and 1.5 Hz).

| High Mass (FAB, M+1): | |
|---|---|
| Calculated C₂₅H₃₃N₂O: | 377.2593. |
| Found: | 377.2598. |

### Example 55 1-[3-[1-(4-Methylbenzyl)-4-piperidinyl]-propyl]-3,4-dihydro-2 (1H)-quinolinone

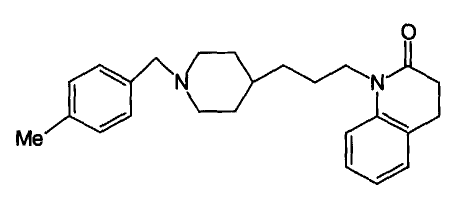

The title compound was synthesized in the same manner as in Example 30, except that 4-methylbenzaldehyde was used instead of 3,4-difluorobenzaldehyde.
¹H NMR (CDCl₃) δ (ppm): 1.18 - 1.33 (5H, m), 1.59 - 1.68 (4H, m), 1.89 (2H, brt, J = 11.0 Hz), 2.32 (3H, s), 2.60 - 2.65 (2H, m), 2.81 - 2.90 (4H, m), 3.44 (2H, s), 3.89 (2H, t, J = 7.8 Hz), 6.96 (1H, brd, J = 8.3 Hz), 6.99 (1H, dt, J = 7.3 and 1.0 Hz), 7.11 (2H, d, J = 7.8 Hz), 7.15 (1H, brd, J = 7.3 Hz), 7.18 (2H, d, J = 7.8 Hz), 7.23 (1H, ddd, J = 8.3, 7.3 and 1.5 Hz).

| High Mass (FAB, M+1): | |
|---|---|
| Calculated C₂₅H₃₃N₂O: | 377.2593. |
| Found: | 377.2598. |

### Pharmacological Test Example 1

### Binding affinity for dopamine D₄ receptor

Cells that express cloned human dopamine D₄ receptor were harvested in an assay buffer (50mM Tris-HCl, pH7.4, containg 120 mM NaCl, 5 mM KCl, 1mM MgCl₂, and 1 mM CaCl₂) and homogenized in a potter type homogenizer. The membrane fraction was centrifuged at 39000 g for 20 min at 4°C. The pellet was suspended in 1 ml of the assay buffer per cells from dish having a diameter of 10 cm and was again homogenated. The aliquots (100µl) was added to the buffer containing [³H]-spiperone of a final concentration of 0.2nM and a test compound to the final assay volume of 300µl, which was then incubated at 37°C for 30 min. The incubation was stopped by rapid filtration on a GF/B filter. Washing with 5 ml of ice-cold 50 mM Tris-HCl (pH 7.4) was then carried out. The radioactivity was measured with a packered liquid scintillation counter. The amount of nonspecific binding was determined in the presence of 1 µM spiperone, and the amount of the specific binding was then determined by subtracting the amount of the nonspecific binding from the total binding. For each compound, IC₅₀ was determined by a regression analysis using a nonlinear least square method. The Ki value was then calculated by the equation of Cheng and Prusoff. The results are summarized in Table 1.

**Table 1**

| Compound of Ex. | Ki (nM) |
|---|---|
| 1 | 7.6 |
| 3 | 18 |
| 10 (A) | 34 |
| 20 | 2.0 |
| 24 | 6.0 |
| 27 | 29 |
| 29 | 5.1 |
| 30 | 5.7 |
| 32 | 7.6 |
| 33 | 1.0 |
| 39 | 5.9 |
| 41 | 1.1 |
| 44 | 2.8 |
| 54 | 7.5 |
| 55 | 1.0 |

These results show that the compounds of the present invention have affinity for dopamine D₄ receptors.

### Pharmacological Test Example 2

### Toxicity test on single administration

Compounds tested in Pharmacological Test Example 1 were intraperitoneally administered to ddY male mice (4 to 6 weeks old, average weight: about 30 g). As a result, at a dose of 32 mg/kg, no articular symptoms developed in all the mice, indicating that these compounds have low toxicity.

## Claims

1. A psychotropic pharmaceutical composition comprising a compound represented by general formula (I) or a pharmaceutically acceptable salt or solvate thereof: 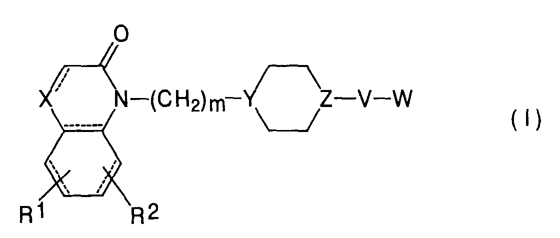 wherein
a solid line with a dotted line represents a single bond or a double bond;
m represents an integer of 1 to 4;
R¹ and R², which may be the same or different, represent a hydrogen atom, a halogen atom, hydroxy, cyano, nitro, trifluoromethyl, -ORa, -SRa, -SORa, -SO₂NRaRb, -NRaRb, -NRaCORb, -NRaCOORb, -CORa, -COORa, or lower alkyl optionally substituted by a halogen atom, wherein Ra and Rb, which may be the same or different, represent lower alkyl optionally substituted by a hydrogen atom or a halogen atom;
X represents CH, CH₂, O, S, SO, or SO₂;
Y and Z, which may be the same or different, represent CH or N;
V represents O or -(CH₂)ₙ- wherein n is an integer of 1 to 4; and
W represents a group selected from the group consisting of groups (i) to (iii): 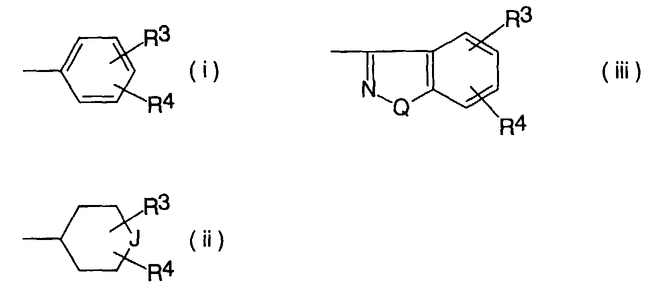 wherein J represents CH₂ or O, Q represents O, S, or NH, and R³ and R⁴, which may be the same or different, represent a hydrogen atom, a halogen atom, cyano, or lower alkyl or lower alkoxy optionally substituted by a halogen atom, or R³ and R⁴ may form a five- or six-membered saturated or unsaturated ring which may contain one or more oxygen, nitrogen, or sulfur atoms together with a carbon atom to which they are attached.

2. A pharmaceutical composition according to claim 1, wherein, in general formula (I), any one of or both Y and Z represent N, V represents -(CH₂)ₙ-, and W represents group (i).

3. A pharmaceutical composition according to claim 1, wherein, in general formula (I), any one ofor both Y and Z represent N, V represents -(CH₂)ₙ-, and W represents group (ii).

4. A pharmaceutical composition according to claim 1, wherein, in general formula (I), any one ofor both Y and Z represent N, V represents -O-, and W represents group (i).

5. A pharmaceutical composition according to claim 1, wherein, in general formula (I), any one ofor both Y and Z represent N, V represents -(CH₂)ₙ-, and W represents group (iii).

6. A pharmaceutical composition according to claim 1, wherein, in general formula (I), R¹ and R² represent a hydrogen atom.

7. A psychotropic pharmaceutical composition comprising a compound selected from the group consisting of the following or a pharmaceutically acceptable salt or solvate thereof:
1-[3-(4-cyclohexanemethyl-1-piperazinyl)-propyl]-2(1H)-quinolinone;
1-[4-(4-cyclohexanemethyl-1-piperazinyl)-butyl]-2(1H)-quinolinone;
1-[3-[4-(3,4-difluorobenzyl)-1-piperaznyl]-propyl]-2(1H)-quinolinone;
1-[4-[4-(3,4-difluorobenzyl)-1-piperazinyl]-butyl]-2(1H)-quinolinone;
1-[3-(4-piperonyl-1-piperazinyl)-propyl]-2(1H)-quinolinone;
1-[4-(4-piperonyl-1-piperazinyl)-butyl]-2(1H)-quinolinone;
1-[3-(4-cyclohexanemethyl-1-piperidinyl)-propyl]-2(1H)-quinolinone;
1-[2-(4-cyclohexanemethyl-1-piperazinyl)-ethyl]-2(1H)-quinolinone;
1-[2-(4-cyclohexanemethyl-1-piperidinyl)-ethyl]-2(1H)-quinolinone,
1-[2-(4-cyclohexanemethyl-1-piperazinyl)-ethyl]-3,4-dihydro-2(1H)-quinolinone;
1-[2-(4-cyclohexanemethyl-1-piperarzinyl)-ethyl]-octahydro-2(1H)-quinolinone;
1-[2-(4-cyclohexanemethyl-1-piperidinyl)-ethyl]-octahydro-2(1H)-quinolinone;
1-[3-(4-cyclohexanemethyl-1-piperidinyl)-propyl]-3,4-dihydro-2 (1H)-quinolinone;
1-[3-(4-cyclohexanemethyl-1-piperidinyl)-propyl]-octahydro-2(1H)-quinolinone;
1-[3-[4-(4-chlorobenzyl)-1-piperidinyl]-propyl]-2(1H)-quinolinone,
1-[3-[4-(2,4-difluorobenzyl)-1-piperidinyl]-propyl]-2(1H)-quinolinone;
1-[3-[4-(3,4-difluorobenzyl)-1-piperidinyl]-propyl]-2(1H)-quinolinone;
1-[3-[4-(4-tetrahydropyran)-1-piperidinyl]-propyl]-2(1H)-quinolinone;
1-[3-[4-(4-methoxybenzyl)-1-piperidinyl]-propyl]-2(1H)-quinolinone;
1-[3-[4-(4-tert-butylbenzyl)-1-piperidinyl]-propyl]-2(1H)-quinolinone;
1-[3-[4-phenoxy-1-piperidlnyl]-propyl]-2 (1H)-quinolinone;
1-[3-[4-(4-chlorophenoxy)-1-piperidinyl]-propyl]-2(1H)-quinolinone;
1-[4-[4-(benzisoxazole-3-methyl)-1-piperazinyl]-propyl]-2(1H)-quinolinone;
1-[3-[4-(2-methoxybenzyl)-1-piperazinyl]-propyl]-2(1H)-quinolinone;
1-[3-(1-cyclohexanemethyl-4-piperidinyl)-propyl]-2(1H)-quinolinone;
1-[3-[1-(3,4-difluorobenzyl)-4-piperidinyl]-propyl]-2(1H)-quinolinone;
1-[3-[4-(4-chlorophenoxy)-1-piperidinyl]-ethyl]-2(1H)-quinolinone;
1-[3-[1-(3,4-difluorobenzyl)-4-piperidinyl]-propyl]-4H-benzo[1,4]oxazin-3-one;
1-[3-[1-(4-fluorobenzyl)-4-piperidinyl]-propyl]-4H-benzo[1,4]oxazin-3-one;
1-[3-[1-(3-fluorobenzyl)-4-piperidinyl]-propyl]-4H-benzo[1,4]oxazin-3-one;
1-[3-[1-(4-chlorobenzyl)-4-piperidinyl]-propyl]-4H-benzo[1,4]oxazin-3-one;
1-[3-[1-(3,4-difluorobenzyl)-4-piperidinyl]-propyl]-3,4-dihydro-2 (1H)-quinolinone;
1-[3-[1-(3,4-difluorobenzyl)-4-piperidinyl]-propyl]-4H-benzo[1,4]thiazin-3-one;
1-[3-[1-(4-fluorobenzyl)-4-piperidinyl]-propyl]-4H-benzo[1,4]thiazin-3-one;
1-[3-[1-(4-chlorobenzyl)-4-piperidinyl]-propyl]-4H-benzo[1,4]thiazin-3-one;
1-[3-[1-(4-trifluoromethylbenzyl)-4-piperidinyl]-propyl]-4H-benzo[1,4]thiazin-3-one;
1-[3-[1-(4-methoxybenyl)-4-piperidinyl]-propyl]-4H-benzo[1,4]thiazin-3-one;
1-[3-[1-(4-methylbenzyl)-4-piperidinyl]-propyl]-4H-benzo[1,4]thiazin-3-one;
1-[3-[1-(2-methylbenzyl)-4-piperidinyl]-propyl]-4H-benzo[1,4]thiazin-3-one;
1-[3-[1-benzyl-4-piperidinyl]-propyl]-3,4-dihydro-2(1H)-quinolinone;
1-[3-[1-(2,4-difluorobenzyl)-4-piperidinyl]-propyl]-3,4-dihydro-2 (1H)-quinolinone;
1-[3-[1-(4-fluorobenzyl)-4-piperidinyl]-propyl]-3,4-dihydro-2(1H)-quinolinone;
1-[3-[1-(4-chlorobenzyl)-4-piperidinyl]-propyl]-3,4-dihydro-2(1H)-quinolinone;
1-[3-[1-(4-trifuoromethylbenzyl)-4-piperidinyl]-propyl]-3,4-dihydro-2 (1H)-quinolinone;
1-[3-[1-(4-methoxybenzyl)-4-piperidinyl]-propyl]-3,4-dihydro-2(1H)-quinolinone;
1-[3-[1-(2-methylbenzyl)-4-piperidinyl]-propyl]-3,4-dihydro-2(1H)-quinolinone,
1-[3-[1-(4-nitrilebenzyl)-4-piperidinyl]-propyl]-4H-benzo[1,4]oxazin-3-one;
1-[3-[1-(4-methoxybenzyl)-4-piperidinyl]-propyl]-4H-benzo[1,4]oxazin-3-one;
1-[3-[1-(4-methylbenzyl)-4-piperidinyl]-propyl]-4H-benzo[1, 4]oxazin-3-one;
1-[3-[1-(4-trifluoromethylbenzyl)-4-piperidinyl]-propyl]-4H-benzo[1, 4]oxazin-3-one;
1-[3-[1-(4-isopropylbenzyl)-4-piperidinyl]-propyl]-4H-benzo[1,4]oxazin-3-one;
1-[3-[1-(4-tert-butylbenzyl)-4-piperidinyl]-propyl]-4H-benzo[1,4]oxazin-3-one;
1-[3-[1-(2-fluorobenzyl)-4-piperidinyl]-propyl]-4H-benzo[1,4]oxazin-3-one;
1-[3-[1-(2-chlorobenzyl)-4-piperidinyl]-propyl]-4H-benzo[1,4]oxazin-3-one;
1-[3-[1-(2-methoxybenzyl)-4-piperidinyl]propyl]-4H-benzo[1,4]oxazin-3-one;
1-[3-[1-(3-methylbenzyl)-4-piperidinyl]propyl]-3,4-dihydro-2(1H)-quinolinone; and
1-[3-[1-(4-methylbenzyl)-4-piperidinyl]propyl]-3,4-dihydro-2(1H)-quinolinone.

8. A pharmaceutical composition according to claim 1, which is used as antipsychotic agents, antianxiety agents, or antidepressants.

9. A method for treating mental diseases, anxiety syndrome, and/or depression, comprising the step of administering an effective amount of a compound represented by general formula (I) according to claim 1 or a pharmacologically acceptable salt or solvate thereof to a mammal.

10. Use of a compound represented by general formula (I) according to claim 1 or a pharmacologically acceptable salt or solvate thereof for the manufacture of psychotropic agents.

11. A compound of general formula (Ia): 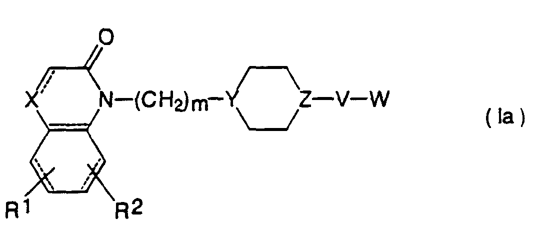 wherein
a solid line with a dotted line represents a single bond or a double bond;
m is an integer of 1 to 4;
R¹ and R², which may be the same or different, represent a hydrogen atom, a halogen atom, hydroxy, cyano, nitro, trifluoromethyl, -ORa, -SRa, -SORa, -SO₂NRaRb, -NRaRb, -NRaCORb, -NRaCOORb, -CORa, -COORa, or lower alkyl optionally substituted by a halogen atom, wherein Ra and Rb, which may be the same or different, represent hydrogen or lower alkyl optionally substituted by a halogen atom;
X represents CH, CH₂, O, S, SO, or SO₂;
Y and Z, which may be the same or different, represent CH or N;
V represents O or -(CH₂)ₙ- wherein n is an integer of 1 to 4; and
W represents a group selected from the group consisting of groups (i) to (iii): 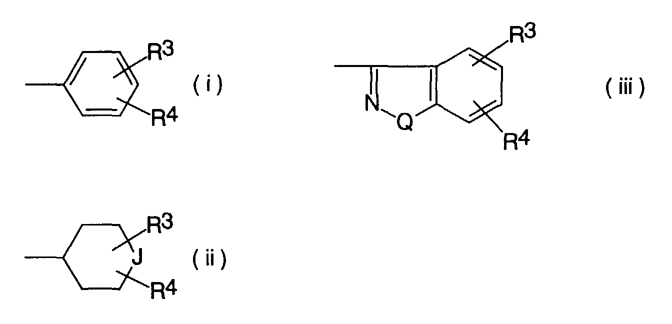 wherein J represents CH₂ or O, Q represents O, S, or NH, and R³ and R⁴, which may be the same or different, represent a hydrogen atom, a halogen atom, cyano, or lower alkyl or lower alkoxy optionally substituted by a halogen atom, or R³ and R⁴ may form a five- or six-membered saturated or unsaturated ring which may contain one or more oxygen, nitrogen, or sulfur atoms together with a carbon atom to which they are attached,
provided that the compounds ofgeneral formula (Ia) wherein X represents CH₂, m is 3, Y represents N, Z represents CH, V represents -CH₂-, and W represents group (i) (wherein R³ and R⁴ represent a hydrogen atom) and wherein X represents CH₂, m is 2, Y represents CH, Z represents N, V represents -CH₂-, and W represents group (i) (wherein R³ and R⁴ represent a hydrogen atom) are excluded.

12. A compound according to claim 11, wherein, in general formula (I), any one of or both Y and Z represent N, V represents - (CH₂)ₙ-, and W represents group (i).

13. A compound according to claim 11, wherein, in general formula (I), any one of or both Y and Z represent N, V represents - (CH₂)ₙ-, and W represents group (ii).

14. A compound according to claim 11, wherein, in general formula (I), any one of or both Y and Z represent N, V represents -O-, and W represents group (i).

15. A compound according to claim 11, wherein, in general formula (I), any one of or both Y and Z represent N, V represents - (CH₂)ₙ-, and W represents group (iii).

16. A compound according to claim 11, wherein, in general formula (I), R¹ and R² represent a hydrogen atom.

17. A compound selected from the group consisting of the following or a pharmaceutically acceptable salt or solvate thereof:
1-[3-(4-cyclohexanemethyl-1-piperazinyl)-propyl]-2(1H)-quinolinone;
1-[4-(4-cyclohexanemethyl-1-piperazinyl)-butyl]-2(1H)-quinolinone;
1-[3-[4-(3,4-difluorobenzyl)-1-piperazinyl]-propyl]-2(1H)-quinolinone;
1-[4-[4-(3,4-difluorobenzyl)-1-piperazinyl]-butyl]-2(1H)-quinolinone;
1-[3-(4-piperonyl-1-piperazinyl)-propyl]-2(1H)-quinolinone;
1-[4-(4-piperonyl-1-piperazinyl)-butyl]-2(1H)-quinolinone;
1-[3-(4-cyclohexanemethyl-1-piperidinyl)-propyl]-2(1H)-quinolinone;
1-[2-(4-cyclohexanemethyl-1-piperazinyl)-ethyl]-2(1H)-quinolinone;
1-[2-(4-cyclohexanemethyl-1-piperidinyl)-ethyl]-2(1H)-quinolinone,
1-[2-(4-cyclohexanemethyl-1-piperazinyl)-ethyl]-3,4-dihydro-2(1H)-quinolinone,
1-[2-(4-cyclohexanemethyl-1-piperazinyl)-ethyl]-octahydro-2(1H)-quinolinone;
1-[2-(4-cyclohexanemethyl-1-piperidinyl)-ethyl]-octahydro-2(1H)-quinolinone;
1-[3-(4-cyclohexanemethyl-1-piperidinyl)-propyl]-3,4-dihydro-2 (1H)-quinolinone;
1-[3-(4-cyclohexanemethyl-1-piperidinyl)-propyl]-octahydro-2(1H)-quinolinone;
1-[3-[4-(4-chlorobenzyl)-1-piperidinyl]-propyl]-2(1H)-quinolinone,
1-[3-[4-(2,4-difluorobenzyl)-1-piperidinyl]-propyl]-2(1H)-quinolinone;
1-[3-[4-(3,4-difluorobenyl)-1-piperidinyl]-propyl]-2(1H)-quinolinone;
1-[3-[4-(4-tetrahydropyran)-1-piperidinyl]-propyl]-2(1H)-quinolinone;
1-[3-[4-(4-methoxybenyl)-1-piperidinyl)-propyl]-2(1H)-quinolinone;
1-[3-[4-(4-tert-butylbenzyl)-1-piperidinyl]-propyl]-2(1H)-quinolinone;
1-[3-[4-phenoxy-1-piperidinyl]-propyl]-2(1H)-quinolinone;
1-[3-[4-(4-chlorophenoxy)-1-piperidinyl]-propyl]-2(1H)-quinolinone;
1-[4-[4-(benzisoxazole-3-methyl)-1-piperazinyl]-propyl]-2(1H)-quinolinone;
1-[3-[4-(2-methoxybenzyl)-1-piperazinyl]-propyl]-2(1H)-quinolinone;
1-[3-(1-cyclohexanemethyl-4-piperidinyl)-propyl]-2(1H)-quinolinone;
1-[3-[1-(3,4-difluorobenzyl)-4-piperidinyl]-propyl]-2(1H)-quinolinone;
1-[3-[4-(4-chlorophenoxy)-1-piperidinyl]-ethyl]-2(1H)-quinolinone;
1-[3-[1-(3,4-diiluorobenzyl)-4-piperidinyl]-propyl]-4H-benzo[1,4]oxazin-3-one;
1-[3-[1-(4-fluorobenzyl)-4-piperidinyl]-propyl]-4H-benzo[1,4]oxazin-3-one;
1-[3-[1-(3-fluorobenzyl)-4-piperidinyl]-propyl]-4H-benzo[1,4]oxazin-3-one;
1-[3-[1-(4-chlorobenzyl)-4-piperidinyl]-propyl]-4H-benzo[1,4]oxazin-3-one;
1-[3-[1-(3,4-difluorobenzyl)-4-piperidinyl]-propyl]-3,4-dihydro-2 (1H)-quinolinone;
1-[3-[1-(3,4-difluorobenzyl)-4-piperidinyl]-propyl]-4H-benzo[1,4]thiazin-3-one;
1-[3-[1-(4-fluorobenzyl)-4-piperidinyl]-propyl]-4H-benzo[1,4]thiazin-3-one;
1-[3-[1-(4-chlorobenzyl)-4-piperidinyl]-propyl]-4H-benzo[1,4]thiazin-3-one;
1-[3-[1-(4-trifluoromethylbenzyl)-4-piperidinyl]-propyl]-4H-benzo[1,4]thiazin-3-one;
1-[3-[1-(4-methoxybenzyl)-4-piperidinyl]-propyl]-4H-benzo[1,4]thiazin-3-one;
1-[3-[1-(4-methylbenzyl)-4-piperidinyl]-propyl]-4H-benzo[1,4]thiazin-3-one;
1-[3-[1-(2-methylbenzyl)-4-piperidinyl]-propyl]-4H-benzo[1,4]thiazin-3-one;
1-[3-[1-benzyl-4-piperidinyl]-propyl]-3,4-dihydro-2(1H)-quinolinone;
1-[3-[1-(2,4-difluorobenzyl)-4-piperidinyl]-propyl]-3,4-dihydro-2 (1H)-quinolinone;
1-[3-[1-(4-fluorobenzyl)-4-piperidinyl]-propyl]-3,4-dihydro-2(1H)-quinolinone;
1-[3-[1-(4-chlorobenxyl)-4-piperidinyl]-propyl]-3,4-dihydro-2(1H)-quinolinone;
1-[3-[1-(4-trifluoromethylbenzyl)-4-piperidinyl]-propyl]-3,4-dihydro-2 (1H)-quinolinone;
1-[3-[1-(4-methoxybenzyl)-4-piperidinyl]-propyl]-3,4-dihydro-2(1H)-quinolinone;
1-[3-[1-(2-methylbenzyl)-4-piperidinyl]-propyl]-3,4-dihydro-2(1H)-quinolinone,
1-[3-[1-(4-nitrilebenzyl)-4-piperidinyl]-propyl]-4H-benzo[1,4]oxazin-3-one;
1-[3-[1-(4-methoxybenzyl)-4-piperidinyl]-propyl]-4H-benzo[1,4]oxazin-3-one;
1-[3-[1-(4-methylbenzyl)-4-piperidinyl]-propyl]-4H-benzo[1, 4]oxazin-3-one;
1-[3-[1-(4-trifluoromethylbenzyl)-4-piperidinyl]-propyl]-4H-benzo[1, 4]oxazin-3-one;
1-[3-[1-(4-isopropylbenzyl)-4-piperidinyl]-propyl]-4H-benzo[1,4]oxazin-3-one;
1-[3-[1-(4-tert-butylbenzyl)-4-piperidinyl]-propyl]-4H-benzo[1,4]oxazin-3-one;
1-[3-[1-(2-fluorobenzyl)4-piperidinyl]-propyl]-4H-benzo[1,4]oxazin-3-one;
1-[3-[1-(2-chlorobenzyl)-4-piperidinyl]-propyl]-4H-benzo[1,4]oxazin-3-one;
1-[3-[1-(2-methoxybenzyl)-4-piperidinyl]-propyl]-4H-benzo[1,4]oxazin-3-one;
1-[3-[1-(3-methylbenzyl)-4-piperidinyl]-propyl]-3,4-dihydro-2(1H)-quinolinone; and
1-[3-[1-(4-methylbenzyl)-4-piperidinyl]-propyl]-3,4-dihydro-2(1H)-quinolinone.
